# EUROPEAN PATENT APPLICATION

(11) **EP 2 989 904 A1**
(43) Date of publication of application: **02.03.2016**
(21) Application number: 14002638.6
(22) Date of filing: 29.07.2014
(51) Int. Cl.: A23L 33/10, A61K 39/00, A61K 36/06, A61P 37/00

(54) **Induction of immunological tolerance using yeast**

(71) Applicant: Heinrich-Heine-Universität, 40225 Düsseldorf (DE)
(72) Inventor: Ernst, Joachim, 40721 Hilden (DE); Bürth, Christoph, 40215 Düsseldorf (DE); Kieseier, Bernd, 40667 Meerbusch (DE); Mausberg, Anne-Kathrin, 41466 Neuss (DE)
(74) Representative: Lahrtz, Fritz

(57) **Abstract**

The present invention relates to induction of immunological tolerance, and in particular to yeasts and compositions comprising yeast cells, respectively, for use in the prevention of an autoimmune disease in a subject, wherein an antigen associated with an autoimmune disease is carried on the surface of said yeast (cells) and wherein said yeast and composition, respectively, is administered orally to said subject.

## Description

### Field of the Invention

The present invention relates to the field of induction of immunological tolerance as well as to the field of prevention and treatment of autoimmune diseases.

### Background of the Invention

Autoimmune diseases affect millions of people worldwide. They can have serious effects on life quality and the lifespan. In autoimmune diseases, the immune system itself is harmful - unlike in most other diseases where the immune system serves to attack foreign intruders. In autoimmune diseases, the immune system loses the ability to differentiate foreign from own molecules of the body. Hence, in case of autoimmune diseases, setting up a treatment is usually much more difficult, because it has to account for or even work against the action of the immune response to prevent or ameliorate the disease.

Examples of well-known autoimmune diseases include multiple sclerosis, rheumatoid arthritis, Crohn's disease, diabetes mellitus type 1, ulcerative colitis, autoimmune uveitis, ankylosing spondylitis, and systemic lupus erythematosus.

Despite intensive research, the exact reasons for the onset of autoimmune diseases are still poorly understood. This is why current therapies of autoimmune diseases mainly focus on the relief of symptoms rather than on a prevention of the underlying biological mechanisms. In particular, strategies for preventing autoimmune diseases are hardly available.

As already apparent from the above listed diseases, autoimmune diseases affect various body components such as the joints or the nervous system.

Autoimmune responses mounted against the nervous system represent the cause of various serious human diseases. It is commonly accepted that specific autoimmune reactions directed against components of myelin sheath or axons result in a chronic inflammation within the central nervous system (CNS), clinically classified as multiple sclerosis. Multiple sclerosis (hereinafter also "MS") is the most common cause of lasting disablement in young grown-ups. It is an inflammatory-mediated disease in which the immune system acts against the human central nervous system (CNS) and destroys myelin sheaths and axons. At present, it is assumed that MS is based on an autoimmune reaction against components of the myelin sheath. One of the presently studied target antigens is myelin oligodendrocytic glycoprotein (MOG). The exact reason for the development of MS is currently still unclear.

In the last years, various partially effective but very cost-intensive therapies for the amelioration of symptoms of MS were developed as, for example, interferon beta, glatirame racetate, natalizumab, fingolimod, alemtuzumab, teriflunomid and dimethyl fumarate.

At present, the possibilities of treating MS and other autoimmune diseases are restricted in that all present therapy strategies act in an immunomodulating manner or even immunosuppressively, but, in doing so, do not provide a disease-causal approach. For example, administered interferons only show a moderate effect, and new substances such as the monoclonal antibodies natalizumab and alemtuzumab are in part associated with significant side effects that require an intensive pharmacovigilance. Also for these reasons, there is a substantial need for effective, but safe and simply administered medicaments for preventing and treating MS and other autoimmune diseases.

Tolerance is a key mechanism to prevent autoimmunity. Several ways of tolerance induction have been deliberated and tested. While central tolerance prevents the development of autoagressive T and B lymphocytes in the thymus and the bone marrow, respectively, peripheral tolerance mainly takes place in the lymph node and the periphery.

Oral tolerance is a special form of peripheral tolerance suppressing cellular and/or humoral immune responses induced by oral administered antigens, taking place in the gut-associated lymphoid tissue. Oral tolerance prevents inflammatory responses to the microbiome and may also have evolved to prevent hypersensitivity reactions to food proteins. It may also be used to prevent autoimmunity by feeding target antigens.

Strategies for generating an antigen-specific tolerance for preventing MS or for its treatment are currently not employed. That this might, in principle, be possible by oral administration of antigenic peptides, was assumed in view of certain experiments in animal models of MS. Presumably, the oral tolerance against MS-specific peptides that occurred in this case was achieved by the uptake from the gastrointestinal tract (e.g. from M cells of the Peyer Plaques) and their processing by associated lymphatic cells.

Yeasts, and among those e.g. the food yeast *Candida utilis* have long since been used as a food additive in humans and animals. *Candida utilis* was recognized as generally safe

(GRAS certificate) by the Food and Drug Administration, FDA. Methods have been developed to generate endogenous and exogenous products by this and other yeasts using recombinant DNA technology. Recently, methods have been developed to also produce heterologous secretory proteins in *C. utilis.* The secretory proteins could effectively be secreted into the culture medium or they were linked to cell wall components in the form of surface presentation (Kunigo et al., 2013).

In particular the use of *C*. *utilis* for the modulation of the immune response was so far not described. Moreover, it was so far only indicated in certain yeasts that an antigen-specific antibody may be triggered by oral administration of bacterial antigens or viral antigens, i.e. foreign antigens (principle of "oral vaccine"). The opposite effect, that is the antigen-specific suppression of an immune response by a surface presenting yeast or another microorganism was, however, not yet described.

In context with the present invention, the inventors have succeeded in providing an advantageous preventive and therapeutic strategy that is oriented at the pathogenesis of an autoimmune disease, which is considered to be safe, has the potential for a high effectiveness and regarded as very cost-saving and simple in its application. The strategy for generating tolerance to avoid autoimmune reactions described herein is considered to be equally applicable to multiple sclerosis and other autoimmune diseases.

Important features of the present invention e.g. include that it has been described for the first time that the feeding with a recombinant microorganism, particularly yeasts, can lead to the generation of tolerance against antigens. In addition, e.g. the MOG epitope was presently not produced in a microorganism, let alone in yeast.

The present invention is considered advantageous since it is expected that it can e.g. successfully be used for preventing and treating, respectively, autoimmune diseases. Moreover, the use of the invention is extremely cost-saving and safe since yeasts can be used that have long since been used in the food industry (GRAS certificate).

### Short description of the Figures

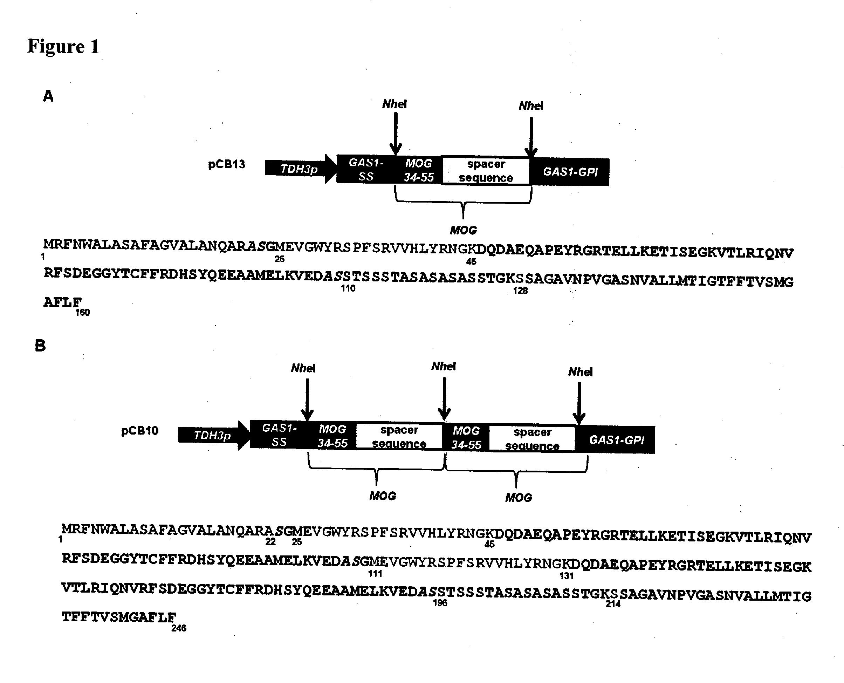

**Figure 1** relates to Mog expression plasmids: **A:** MOG expression unit and amino acid sequence of the Gas1-Mog fusion protein. Single MOG sequence was cloned between *GAS1* signal sequence and GAS1-GPI sequence. **B:** Tandem MOG sequence and amino acid sequence of Gasl-Mog-Mog fusion protein. The Mog₃₄₋₅₅ sequence, including the immunogenic Mog35-55 sequence, (grey bar) is linked to the Gas1-GPI sequence (dark grey) with a non-immunogenic spacer sequence (white bar). Amino acid sequences are shown below the expression units. Gas1 signal peptide is highlighted at the N-terminus of each sequence, immunogenic MOG₃₅₋₅₅ ("ME...GK") are also highlighted; spacer sequence is in black and the Gas1-Gpi sequence at the C-terminus of each sequence is emphasized. *Nhe*I restriction site (*italics*) and the potential ω amino acid (cf. light letters at position 128 and 214, respectively) are also indicated.

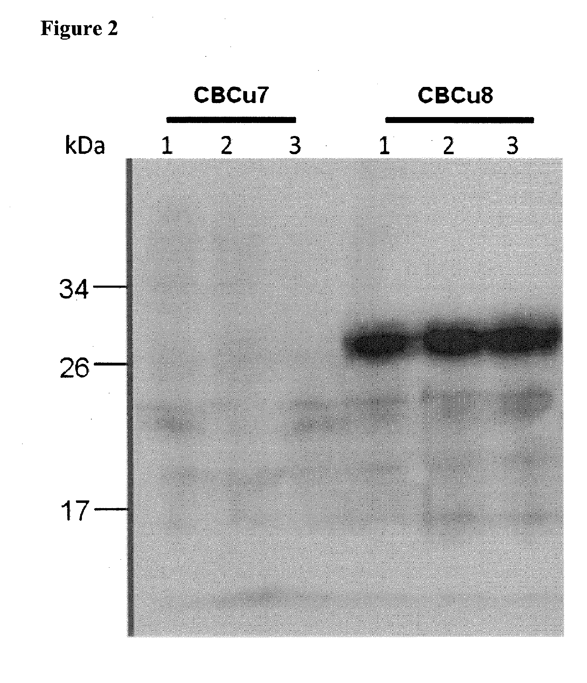

**Figure 2** relates to MOG expression. Three transformants of CBCu7 and CBCu8 were grown in YPD media for 72 h. Cell-associated proteins were separated by SDS-PAGE (4-20 % acrylamide). MOG was detected by immunoblotting using an anti-MOG-antibody.

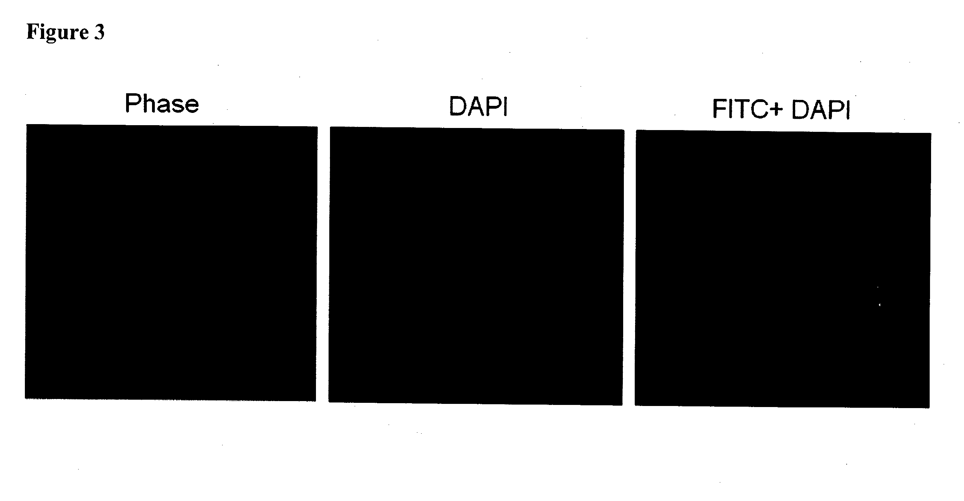

**Figure 3** shows immunofluorescence of CBCu8: Cell wall associated MOG peptide was detected on the surface of non-permeabilized CBCu8 cells with a primary anti-MOG-antibody and a secondary FITC-coupled antibody. Nuclear DNA was stained with DAPI. Cells were grown for 72 h in YPD.

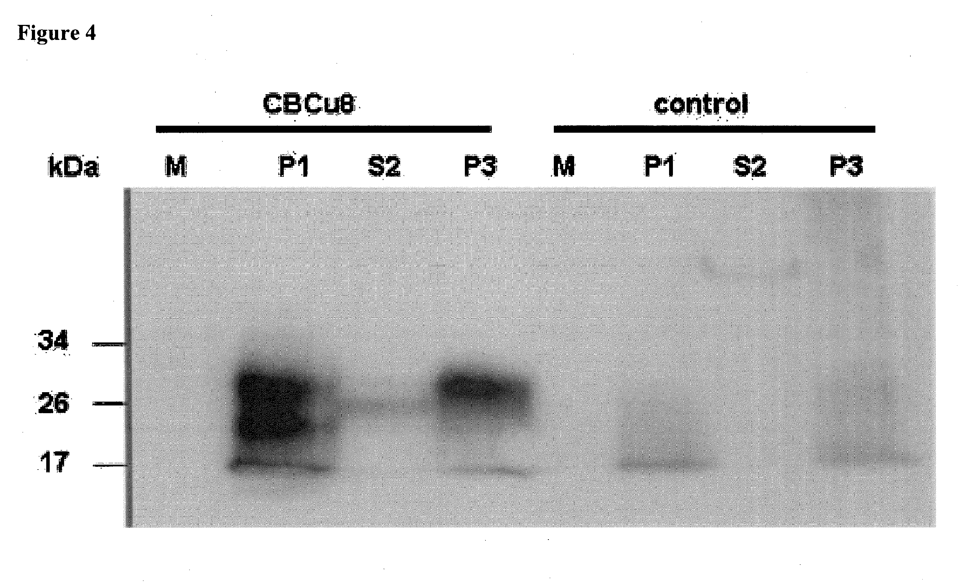

**Figure 4** shows MOG peptide in cell fractions of CBCu8. Cell fractions of stationary CBCu8 cells were separated by differential centrifugation. Equimolar amounts of samples were loaded on a SDS-PAGE (4-20 % acrylamide). M = culture medium; P1 = cell debris cell wall; P3 = enriched ER fraction. Pellet P1 was treated with Zymolyase (10 U) for 1 h, centrifuged and the supernatant (S2) was concentrated (10x) and loaded on the gel.

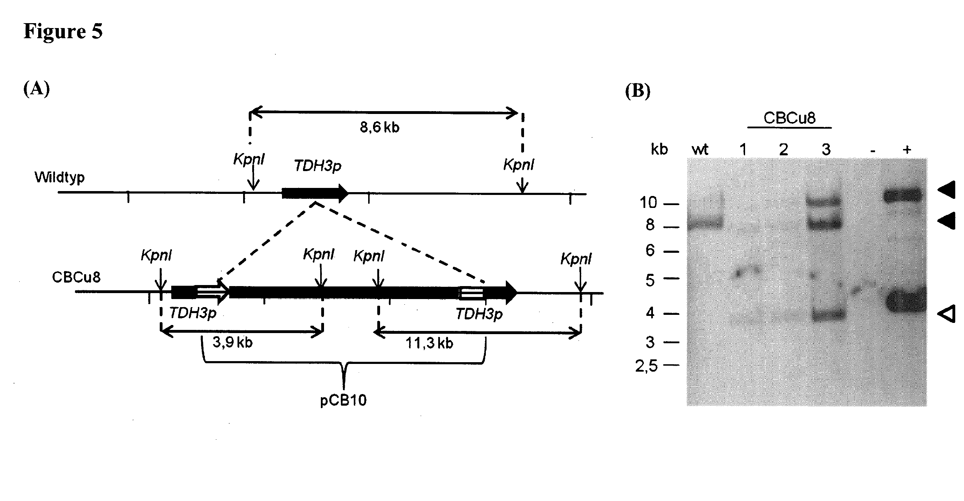

**Figure 5** depicts chromosomal plasmid integration locus and Southern Blot analysis. **(A)** Chromosomal *TDH3* promoter locus of *C. utilis.* Restriction of chromosomal DNA with *Kpn*I results in an 8.6 kb fragment in the wild-type strain. A single integration of plasmid pCB10 leads to 3.9 kb and 11.3 kb fragments. **(B)** *Kpn*I-treated genomic DNA of three CBCu8 transformants and of *C. utilis* wild-type was analysed on a Southern Blot. The plasmid was detected with a *TDH3p* probe. Plasmid pMC1 (Kunigo *et al.,* 2013) was used as positive control. Chromosomal *TDH3p* band is indicated by the black triangle, the 11.3 kb and the 3.9 *TDH3p* plasmid fragments are indicated by a grey triangle and transparent triangle, respectively.

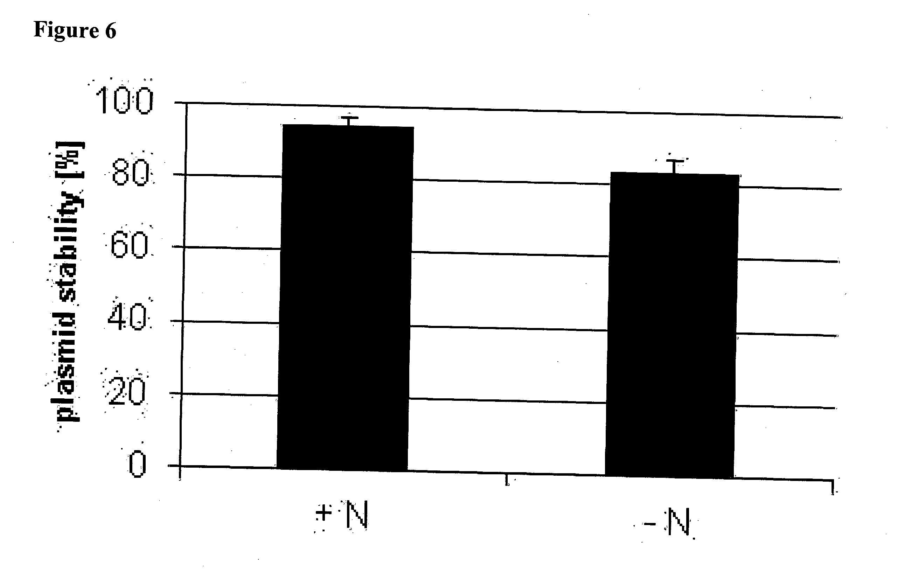

**Figure 6** relates to plasmid stability of CBCu8. MOG transformants were incubated for 50 generations with (+N) or without (-N) Nourseothricin in YPD. Afterwards, cells were selected on YPD agar containing Nourseothricin (10 µg/ ml). Plasmid stability was calculated for 100 cells. Three biological replicates were tested and standard deviations are shown.

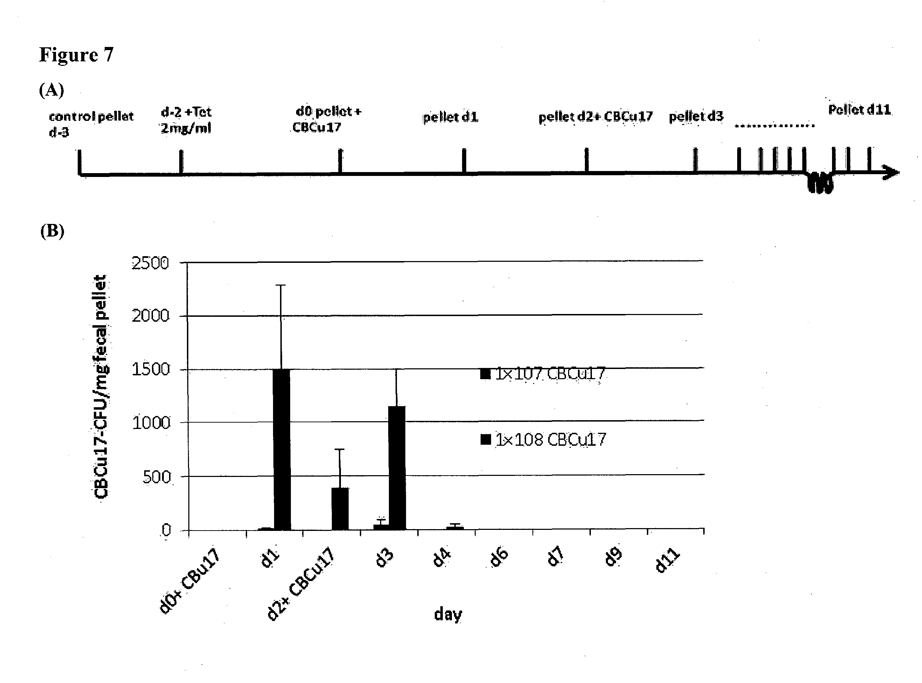

**Figure 7** relates to mouse colonization of CBCu17. (A) Timeline of colonization. A control pellet was collected three days (d-3) before the first *C. utilis* feeding to quantify the natural microbiome. Two days (d-2) before the first *C. utilis* feeding tetracycline (1 mg/ml) was added to the drinking water. On day 0 (d0) another fecal pellet was collected and one group (n=5) was administered with 1x10⁷ CBCu17 cells another group with 1x10⁸ cells. Fecal pellets were collected every 24 hours and on day 2 (d2) mice were fed with CBCu17 cells again. On day 11 the experiment was stopped. (B) The collected fecal pellets were plated out on YPD agar plates containing ampicillin (100 µg/ml) and Nourseothricin (10 µg/ml) and colony forming units were counted. Mean values of three measurements and standard deviations are shown.

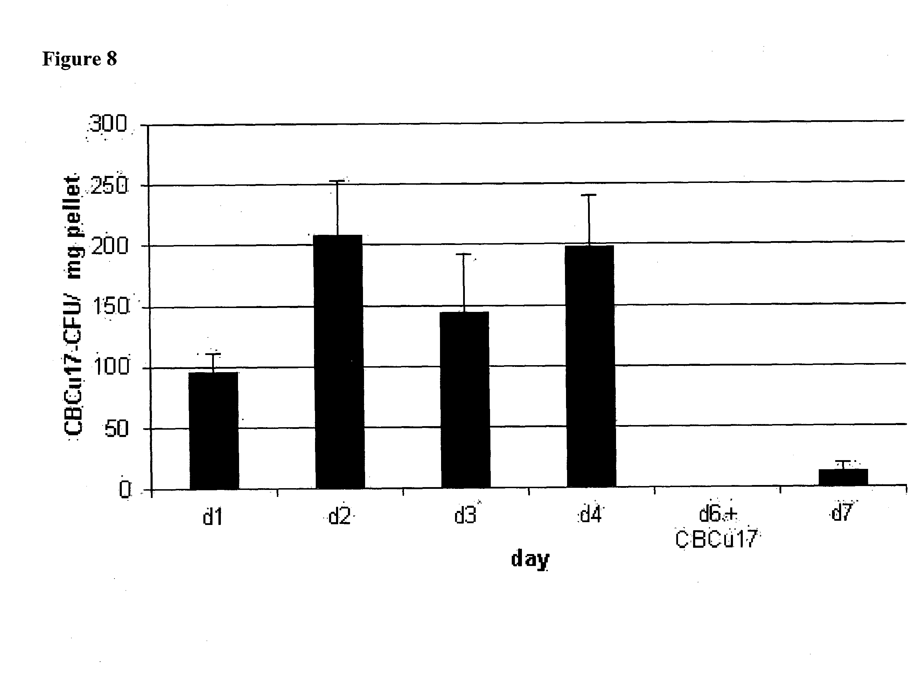

**Figure 8** relates to continuous feeding experiments: One group (n=4) was fed with 1x10⁸ CBCu17 cells for 5 days (d0-d4) every 24 h. On day 5 mice received no CBCu17 cells and on day 6 feeding was started again. To compare CFU between the mice, ratios of CFU to the fecal pellet mass was calculated. Mean values of three measurements and standard deviations are shown.

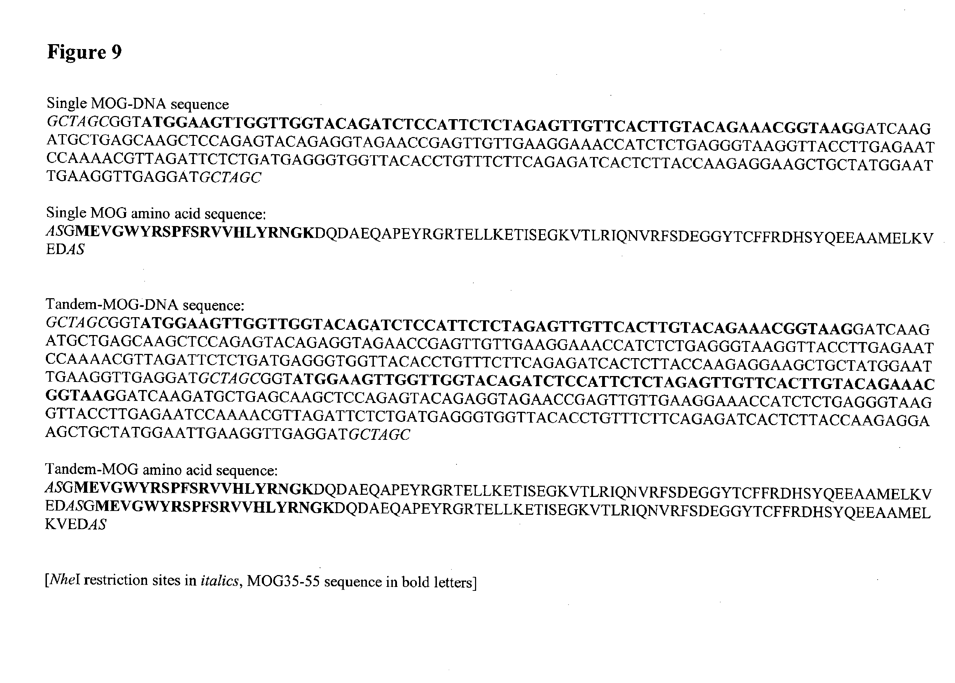

**Figure 9** shows certain MOC sequences in context with the invention.

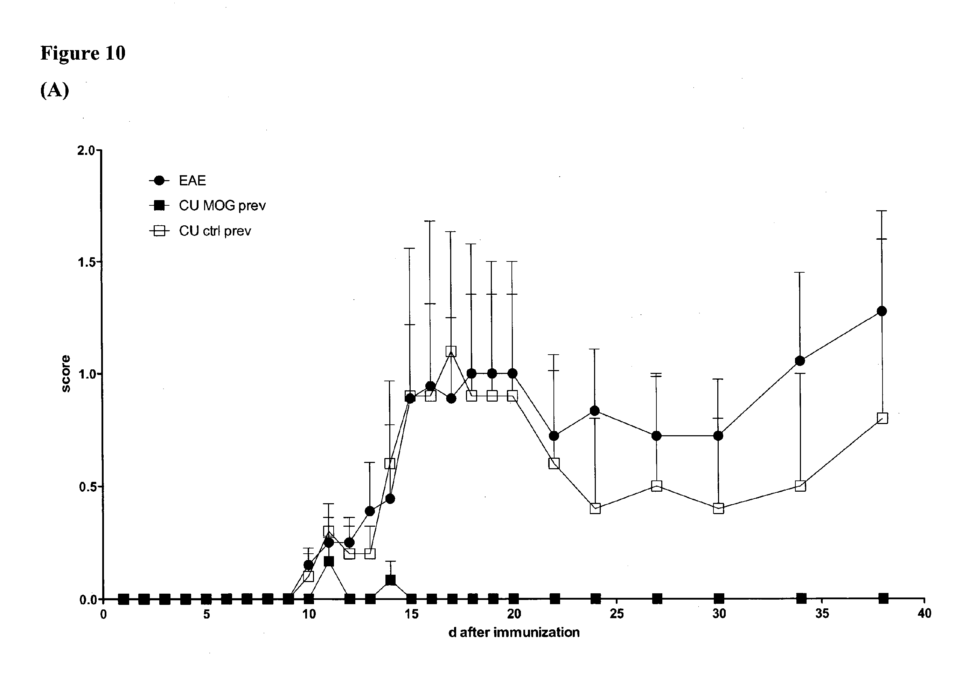

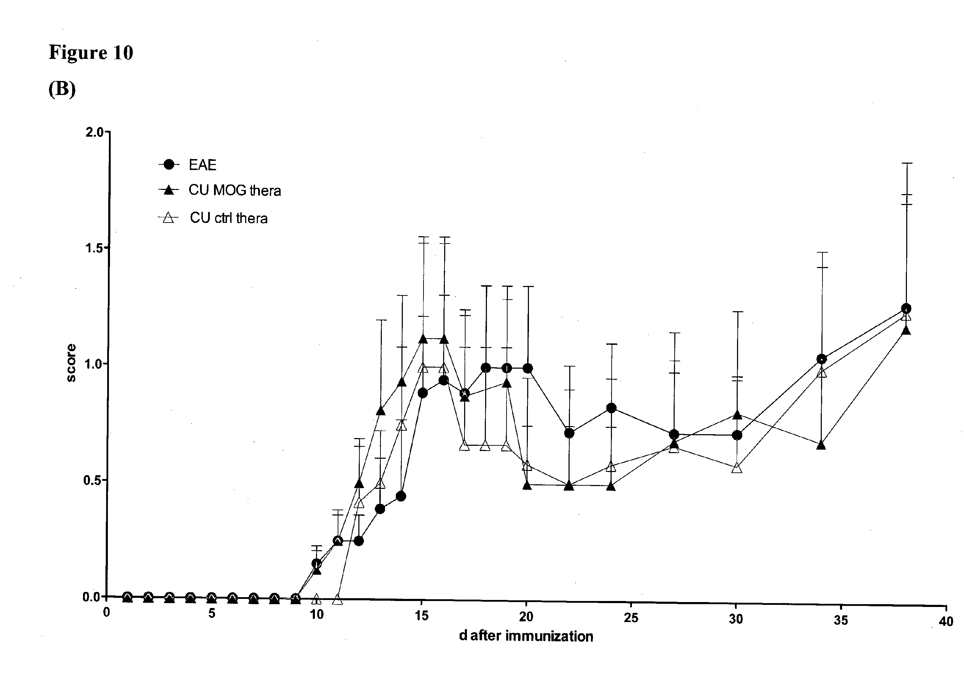

**Figure 10** shows preventive and therapeutic efficacy of CU MOG to reduce the clinical course of EAE. To test the preventive (A) as well as therapeutic potential (B) of CU MOG C57BL/6 animals (six per group) were immunized and 1.5 x 10⁸ Candida were applied daily by oral gavage either 7 days prior (A) or 10 days after immunization (B) with start of the first clinical signs of EAE. Control animals received 1.5 x 10⁸ wild type *Candida utilis* or left untreated. Clinical scoring was performed at indicated time points. 0 = no clinical signs; 1 = tail paralysis; 2 = hind limb paresis; 3 = hind limb paralysis; 4 = fore limp paresis; 5 = death. EAE = immunized mice, untreated; prev = preventive; thera = therapeutic; CU ctrl = wild type *Candida utilis;* CU MOG = CU expressing MOG35-55.

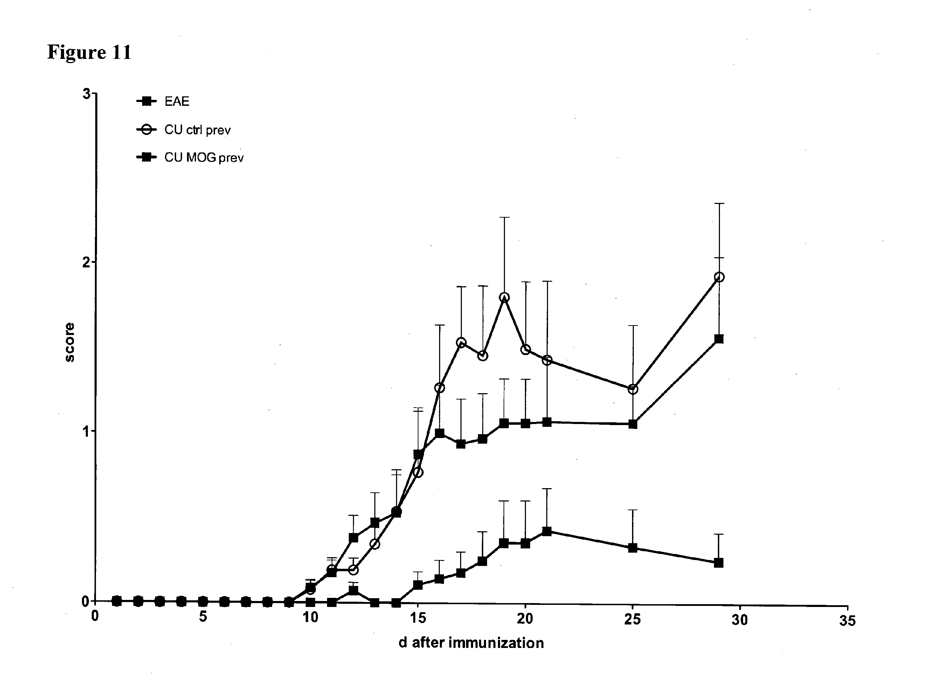

**Figure 11** depicts preventive efficacy of CU MOG on the clinical course of EAE. Mean clinical score of three independent experiments testing the preventive potential of CU MOG. In three independent experiments animals were immunized and 1.5 x 10⁸ Candida were applied daily by oral gavage for 7 days prior immunization. Control animals received 1.5 x 10⁸ wild type *Candida utilis* or left untreated. Clinical scoring was performed at indicated time points. 0 = no clinical signs; 1 = tail paralysis; 2 = hind limb paresis; 3 = hind limb paralysis; 4 = fore limp paresis; 5 = death. EAE = immunized mice, untreated; prev = preventive; CU ctrl = wild type *Candida;* CU MOG = CU expressing MOG35-55.

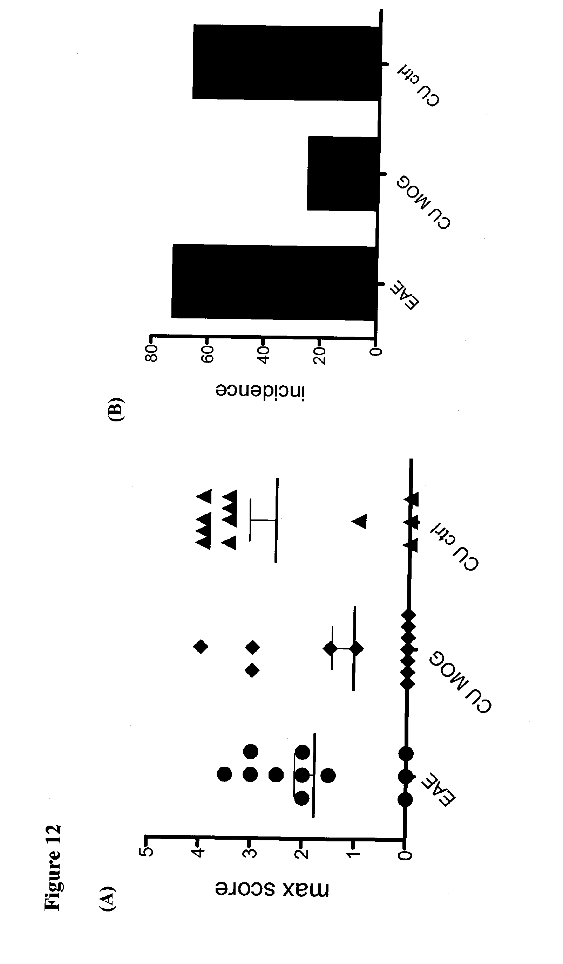

**Figure 12** relates to maximal disease progression and incidence of EAE. Maximal score (A) and incidence (B) of EAE after feeding of the animals in the indicated groups. Max score = maximum score at peak of the disease, EAE = immunized mice, untreated; CU ctrl = wild type Candida; CU MOG = CU expressing MOG35-55.

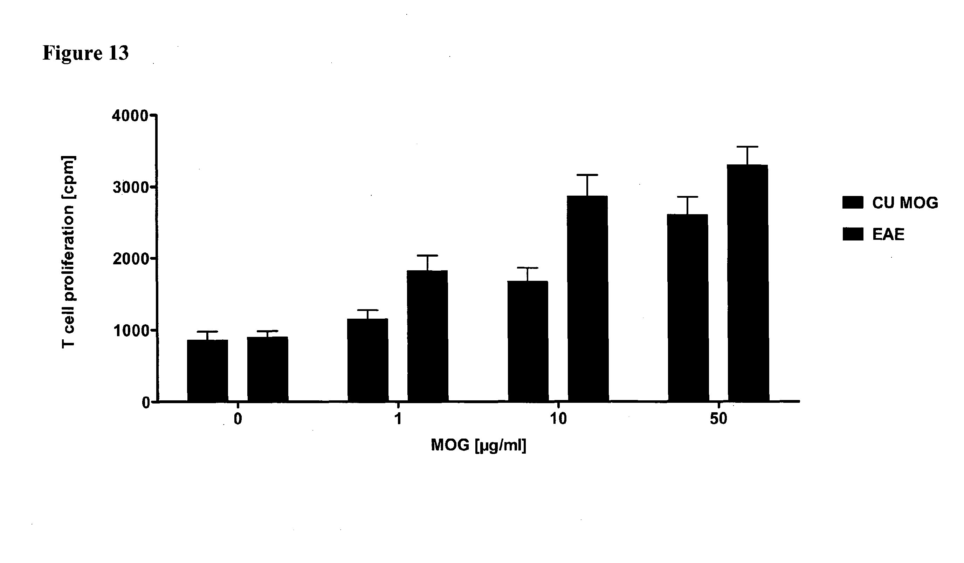

**Figure 13** depicts antigen specific T cell proliferation in CU MOG treated animals. T cell proliferation in response to re-challenge with MOG35-55 in the indicated concentrations. Spleen cell proliferation was measured by ³H-thymidine incorporation at day 15 of EAE in the presence of increasing concentrations of MOG35-55 (0-50 µg/ml) in quadruplicates. Depicted is the SEM of six mice per group.

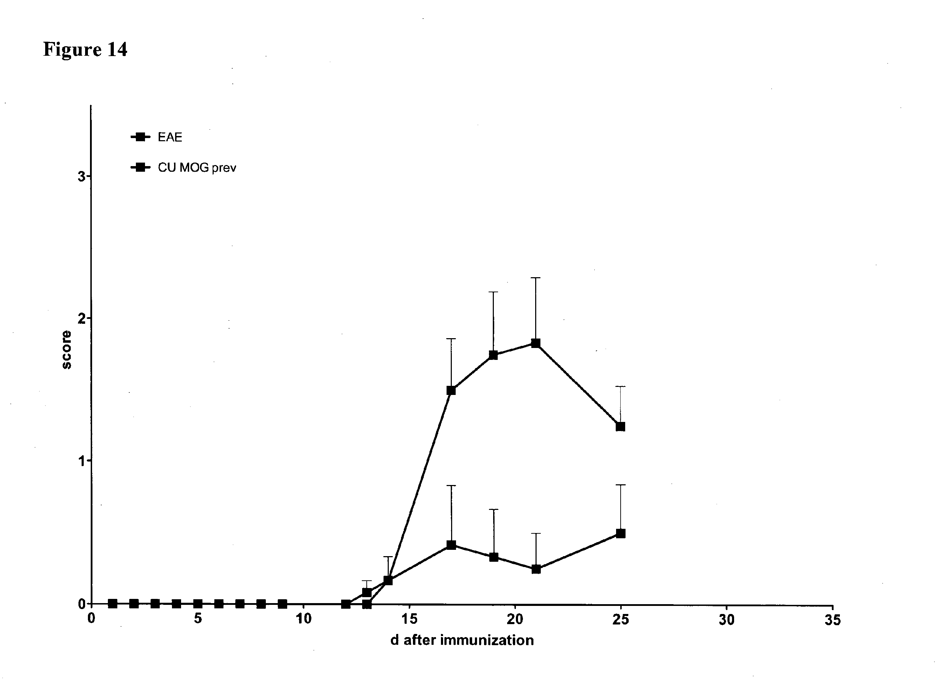

**Figure 14** depicts preventive efficacy of CU MOG in the absence of tetracycline and glucose. To test the preventive potential of CU MOG animals (six per group) were immunized and 1.5 x 10⁸ Candida were applied daily by oral gavage seven days prior immunization with start of the first clinical signs of EAE. Clinical scoring was performed at indicated time points. 0 = no clinical signs; 1 = tail paralysis; 2 = hind limb paresis; 3 = hind limb paralysis; 4 = fore limp paresis; 5 = death. EAE = immunized mice, untreated; prev = preventive; CU MOG = CU expressing MOG35-55.

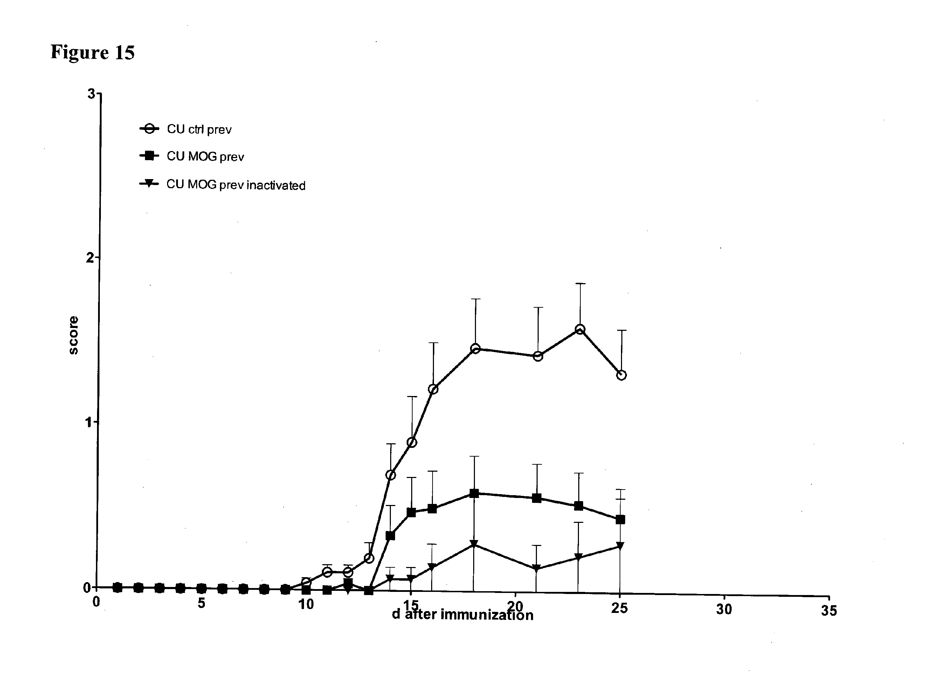

**Figure 15** depicts preventive efficacy of heat inactivated CU MOG on the clinical course of EAE. Mean clinical score of testing the preventive potential of CU MOG heat-inactivated. Ten animals per group were immunized and 1.5 x 10⁸ heat inactivated CU MOG were applied daily by oral gavage for 7 days prior immunization. Control animals received 1.5 x 10⁸ wild type *Candida utilis* or living CU MOG. Clinical scoring was performed at indicated time points. 0 = no clinical signs; 1 = tail paralysis; 2 = hind limb paresis; 3 = hind limb paralysis; 4 = fore limp paresis; 5 = death. prev = preventive; CU ctrl = wild type *Candida;* CU MOG = CU expressing MOG35-55; CU MOG inactivated = CU expressing MOG35-55 heat inactivated.

### Summary of the Invention

In the present invention, a yeast or a composition comprising yeast cells is/are used in the prevention and/or treatment of an autoimmune disease.

Generally, the invention relates to subject-matter as defined in the appended claims.

In particular, the present invention relates to the use of yeast carrying an antigen associated with an autoimmune disease on its surface in the prevention of an autoimmune disease in a subject.

The present invention e.g. relates to a yeast for use in the prevention of an autoimmune disease in a subject, wherein said yeast carries an antigen associated with an autoimmune disease on its surface, and wherein said yeast is administered orally to said subject.

The present invention also relates to a composition comprising yeast cells for use in the prevention of an autoimmune disease in a subject, wherein said yeast cells carry an antigen associated with an autoimmune disease on their surface, and wherein said composition is administered orally to said subject.

### Detailed Description of the Invention

The present invention e.g. aims at preventing the development of autoimmunity directed against an antigen associated with an autoimmune disease, such as against MOG (and therefore against the CNS), and in doing so inhibits the generation and propagation, respectively, of autoimmune diseases, such as multiple sclerosis. In such a preventive method, a specifically constructed yeast (such as *Candida utilis*) is orally administered. As a result, an immunological tolerance for the antigen (such as the MOG antigen) is achieved.

In experiments performed in context with the present invention, a peptide of the myelin oligodendrocyte glycoprotein (MOG) of *C. utilis* was used for surface presentation to examine whether an immune response may be triggered or suppressed, respectively, with modified living *C. utilis* cells. The MOG protein is a structural protein of the myelin sheath of the nerve cells and at the same time represents a strong auto-antigen against which auto-antibodies can be formed. These MOG auto-antibodies can lead to a demyelination of nerve cells in multiple sclerosis. Normally, during the ripening of the immune system in humans, a tolerance originates for this antigenic feature of MOG. It was shown that by injection of relatively high concentrations of MOG, that experimental auto-immune encephalomyelitis (EAE) can be triggered and observed in mice. EAE is very similar to (human) multiple sclerosis and it was therefore examined whether a feeding of a MOG-presenting *C. utilis* strain can suppress an immune response against the MOG peptide. This approach is also based on the consideration that repeated low doses of an antigen may not stimulate antibody production but, as opposed to that, suppress it.

With the resulting and various further findings, the present invention, which is e.g. described in the appended claims, has been completed.

Accordingly, in a first aspect, the present invention relates to a yeast for use in the prevention of an autoimmune disease in a subject, wherein said yeast carries an antigen associated with an autoimmune disease on its surface, and wherein said yeast is administered orally to said subject.

Generally, yeasts, and particularly also yeasts suitable for use in a subject, are known to the skilled person. In the present invention, the term "a yeast" is not particularly limited. It includes reference to one yeast and to more than one yeast. The term also includes reference to yeast cells (and, hence, may e.g. be replaced by the term "yeast cells"), such as to more than one yeast cell, such as to a number of yeast cells. The term may also refer to a particular yeast strain and, hence, also includes reference to a yeast from a particular yeast strain. It also includes reference to yeast cells, such as to more than one yeast cell, such as to a number of yeast cells, from a particular yeast strain. Herein, the yeast (cells) may be of the same yeast strain. In addition, also a mixture of yeast cells from various strains may be employed. In certain embodiments herein, a number of yeast cells refers to from about 1x10² to about 1x10¹⁵ cells, such as from about 1x10³ to about 1x10¹² cells, such as from about 1x10⁴ to 1x10¹⁰ cells. Further (preferred) embodiments for such numbers of yeast cells correspond to those described elsewhere herein, particularly in context with dosages / dosage ranges.

Preferably herein, the yeast is a food yeast. Preferably, said food yeast is suitable for use in human nutrition and/or animal feeding, preferably in human nutrition. Preferably, the yeast herein is approved for use in food products and/or food additives, preferably human food products and/or human food additives.

### In certain embodiments herein, said yeast is selected from the group consisting of Candida utilis, Pichia jadinii, Pichia stipitis, Pichia pastoris, Hansenula polymorpha, Saccharomyces cerevisiae. Preferably herein, the yeast is Candida utilis.

As already indicated above, autoimmune diseases are well-known to the skilled person. In the present invention, the term "autoimmune disease" is not particularly limited. Hence, in certain embodiments, the term autoimmune disease includes the term as used in the art.

Preferably herein, an autoimmune disease is a disease or disorder in which a subject produces an adverse immune response against an own body component. Said body component may also be referred to as self-antigen or auto-antigen.

As will be further described herein below, the said body component may correspond to or be included in an antigen for use in the present invention (cf. an antigen associated with an autoimmune disease). In addition, the latter antigen for use in the present invention may also be derived from such a body component and may e.g. comprise or consist of one or more fragments of such a body component.

As used herein, the term "autoimmune disease" includes "autoimmune disorders", "immunologic disorders", "immune-related diseases", and "immune-related disorders".

In certain embodiments herein, the autoimmune disease is selected from the group consisting of Addison's disease, alopecia, ankylosing spondylitis, autoimmune hepatitis, autoimmune uveitis, colitis ulcerosa, Crohn's disease, diabetes mellitus type 1, early onset diabetes mellitus, Grave's disease, Guillain-Barré-Syndrom, hypoparathyroidism, hypopituitarism, hypothyroidism, lupus erythematodes, multiple sclerosis, myocardititis, pemphigus vulgaris, pernicious anemia, polyglandular failure, polymyosititis, premature ovarian failure, rheumatoid arthritis, sclerodermia, Sjögren's disease, systemic lupus erythematosus, ulcerative colitis, uveitis, and vitiligo.

Preferably, the autoimmune disease is selected from the group consisting of multiple sclerosis, rheumatoid arthritis, Crohn's disease, diabetes mellitus type 1, ulcerative colitis, autoimmune uveitis, ankylosing spondylitis, systemic lupus erythematosus, and Guillain-Barre-Syndrom. More preferably, said autoimmune disease is multiple sclerosis.

Generally, in the present invention, a "prevention" of a disease is not particularly limited. Hence, in certain embodiments, the term prevention corresponds to the term as used in the art. Generally, the exact nature of such preventing may depend on the particular disease.

As used herein, the prevention of an autoimmune disease in a subject may generally refer to preventing a subject from the development of such disease.

As used herein, the prevention of an autoimmune disease also includes preventing of disease recurrence. As used herein, the prevention of an autoimmune disease in a subject also includes preventing deterioration of a disease after it has previously improved. Generally, such previous improvement may be spontaneously or due to any treatment.

In particular, as used herein, the "preventing" of a disease herein includes cases, where one or more symptoms are prevented in a subject that has not shown symptoms of said disease before. As used herein, the "preventing" of a disease herein includes cases, where one or more symptoms are prevented in a subject that has not been diagnosed with the said disease before. As used herein, the "preventing" of a disease herein includes cases, where one or more symptoms are prevented in a subject that has not suffered from the said disease before. In addition, it is intended herein that the "preventing" of a disease includes cases, where one or more symptoms are prevented in a subject, in which such disease has already been triggered. Symptoms may or may not (yet) be visible. In addition, as indicated above, it is intended herein that the "preventing" of a disease also includes preventing disease recurrence. Hence, such "preventing" is also envisaged herein to include cases, where one or more symptoms are prevented in a subject, in which a given disease has previously improved, e.g. spontaneously or via any treatment. Likewise, such "preventing" e.g. also relates to cases, where one or more symptoms are prevented in a subject that has had a given disease before, but has previously successfully been treated, such that e.g. disease symptoms have disappeared or have been ameliorated.

Generally herein, some preferred embodiments relate to a prevention in context with previous disease amelioration, such as previous amelioration of (one or more) symptoms of an autoimmune disease. Accordingly, in certain embodiments a subsequent deterioration of a previous amelioration is prevented in context with an autoimmune disease.

Generally, as used herein, the said "prevention" may also refer to maintaining a negative diagnosis for a given autoimmune disease. Accordingly, certain further embodiments herein correspond to the above embodiments, where the term "one or more symptoms are prevented" is replaced by the term "a negative diagnosis is maintained".

Moreover, a prevention herein also includes cases of a combined treatment involving a yeast (or composition) of the present invention and one or more additional agents, where the use of said yeast (or composition) improves the treatment with the said additional agent(s), e.g. by preventing subsequent deterioration (e.g. a relapse).

Generally, and in light of the above, also the following applies: As used herein, the said "prevention" of an autoimmune disease is envisaged to include the prevention of the onset of said disease. It also includes prevention of symptoms of said disease. It also includes prevention of the triggering of said disease. Accordingly, in certain embodiments herein, an autoimmune disease is prevented in a subject before onset of said disease. In certain embodiments herein, an autoimmune disease is prevented in a subject, in which the disease has not been triggered. In certain embodiments herein, an autoimmune disease is prevented in a subject that does not show symptoms of said disease. Furthermore, in certain embodiments herein, an autoimmune disease is prevented in a subject that does not suffer from said disease. In certain embodiments herein, an autoimmune disease is prevented in a subject that has not been diagnosed with the said disease. In certain embodiments herein, an autoimmune disease is prevented in a subject that has been diagnosed with an autoimmune disease, but does not show symptoms of said disease.

As used herein, the term "antigen associated with an autoimmune disease" is not particularly limited, either, and it will easily be understood by a skilled person. Such antigen for use in context with the invention includes an auto-antigen (or self-antigen, respectively, such as the above-described body component), to which a subject having an autoimmune disease produces an adverse immune response. Hence, an antigen for use in context with the invention includes an antigen (e.g. a self-antigen or as an equivalent term herein an auto-antigen) to which an adverse immune response is produced in an autoimmune disease, and hence may e.g. be a body component of the subject as described above.

Accordingly, an antigen associated with context with an autoimmune disease used herein may be an antigen causing the said autoimmune disease.

Moreover, an antigen associated with an autoimmune disease used herein may be an antigen to which an adverse immune response is produced in an animal model for a given autoimmune disease. Hence, in any case, it is easily possible to test for production of an adverse immune response of a given antigen associated with an autoimmune disease. Particular examples of above (self-)antigens associated with an autoimmune disease include, but are not limited to MOG, MBP, and PLP in case of multiple sclerosis; keratin, collagen and citrullinated peptides/proteins (cf. ACPA) in case of rheumatoid arthritis; glutamic decarboxylase (GAD proteins, e.g. GAD65), insulin, insulin receptor, heat shock protein 60 (Hsp60), IAA, ICA, HLAD, and IA-2 in case of diabetes mellitus type 1; flagellins, I2, porin C, ASCA, and ANCA in case of Crohn's Disease; ANCA in the case of ulcerative colitis.

Moreover, in context with the present invention, the term "antigen associated with an autoimmune disease" also includes antigens that are derived from such self-antigens (or body components, cf. above). For example, an antigen derived from a self-antigen includes an antigen that comprises one or more fragments of a self-antigen. In addition, such antigen for use herein also includes antigens which comprise more than one self-antigen. Likewise, an antigen herein also includes antigens which comprise several fragments of self-antigens, such as two, three, four, five or six fragments thereof. Said fragments may be the same or different fragments. Hence, the antigen may e.g. comprise repeated units of self-antigens. Hence, the antigen may e.g. comprise repeated units of fragments of a self-antigen. Moreover, an antigen derived from a self-antigen generally also includes variants of such self-antigens, such as variants having one or more mutations, deletions and/or additions as compared to said self-antigen. Likewise, it is envisaged that also the said fragments of a self-antigen may comprise one or more mutations, deletions and/or additions as compared to an original fragment.

As to the use of fragments of (self-)antigens in the present invention, the skilled person will readily appreciate that a preferred choice of said fragments may e.g. depend on the immunogenicity of said fragments - and that fragments of a (self-)antigens that have been shown to be or are likely to be responsible for the adverse immune response in the disease are among preferred embodiments. In any case, as apparent from the above, the antigens associated with an autoimmune disease for use in the invention may not be natural (self-)antigens associated with an autoimmune disease (cf. above).

As used herein, the expression that an antigen is "carried on the surface" of a yeast (or yeast cells, respectively) will be readily understood by the skilled person, particularly since surface display of proteins on cell surfaces, and especially also on yeast cell surfaces is well within the skilled person's knowledge. Herein, the said expression includes reference to expression of said antigen as a cell surface protein or membrane protein, respectively. Said antigen may e.g. be an integral or peripheral surface protein. The expression also includes expression of the antigen as part of a (e.g. chimeric) membrane protein of the said yeast (cells). The latter membrane protein may be an integral or peripheral surface protein, as well. The antigen may also be otherwise aggregated and/or associated with the cell surface or cell membrane, respectively. Generally, said antigen carried on the surface of the yeast (cells) may be non-covalently or covalently linked to the cell surface of the yeast - e.g. to one or more cell surface components. Accordingly, said expression includes that the antigen is covalently linked to a surface component, such as a surface protein, of the yeast. Said expression also includes that the antigen is non-covalently linked to the yeast's cell surface, e.g. to one or more cell surface components. As already indicated above, means for achieving cell surface expression in yeast are well-known to the skilled person. Exemplary non-limiting examples include the use of signal sequences, and/or anchor molecules (such as an GPI anchor), and/or PIR proteins and/or disulfide bound proteins. Moreover, the skilled person will readily appreciate that successful surface expression may easily be tested / verified e.g. by labelling experiments, such as experiments similar to those used in the present examples.

Also considered in context with the present invention are embodiments, in which the said antigen associated with an autoimmune disease is (alternatively or additionally) only partly and/or temporarily associated with the surface, e.g. in context of its secretion by the said yeast (cells). Hence, (alternative or additional) secretion of the said antigen associated with an autoimmune disease is also contemplated herein, such as in context of embodiments in which the yeast (cells) is part of a liquid composition for oral administration, which comprise(s) such secreted antigens. Accordingly, certain general further embodiments herein correspond to the above embodiments, where the term "carries an antigen ... on its/their/the surface" and suchlike terms is/are replaced by the term "secrete(s) an antigen ..." and suchlike terms.

Generally, it is not required according to the present invention that all of the used yeast (cells) carries/carry such antigen on the surface. Instead, it is e.g. sufficient that more than 10%, preferably more than 25%, preferably more than 33% of the used yeast (cells) carries/carry same on the surface. Preferably, the majority of the used yeast (cells) carries/carry same on the surface. Preferably, at least 60%, preferably at least 70%, preferably at least 80%, preferably at least 90% of the employed yeast (cells) carries/carry same on the surface.

As used herein, a "subject" is not particularly limited. In certain embodiments, said subject is a non-human animal or a human. In certain embodiments, said subject is a mammal. Preferably herein, the subject is a human. In certain embodiments, said subject is selected from the group consisting of cats, chicken, cows, dogs, horses and pigs. In a non-limiting embodiment, the subject is pre-disposed to developing an autoimmune disease. In a non-limiting embodiment, the subject is a subject in which it is likely, e.g. due to genetic or environmental factors, that an autoimmune disease will break out.

The administration route of an "oral" administration is well-known to the skilled person. Herein, an "oral" administration is not particularly limited, as long as the uptake occurs via the mouth. Oral administration of yeast (cells) in accordance with the present invention may e.g. occur in liquid or solid form. Examples of oral administration of yeast (cells) in liquid form include, but are not limited to suspensions, such as suspended powders, sirups, and the like. Examples of oral administration of yeast (cells) in solid form include, but are not limited to tablets, pills, capsules, granules, and the like.

In certain embodiments of the first aspect, particularly wherein the said autoimmune disease is multiple sclerosis, said antigen is selected from the group consisting of myelin oligodendrocyte glycoprotein (MOG), myelin basic protein (MBP), proteolipid protein (PLP), and fragments thereof. Alternative, said antigen may comprise a protein selected from the group consisting of myelin oligodendrocyte glycoprotein (MOG), myelin basic protein (MBP), proteolipid protein (PLP), and fragments thereof.

Generally, in embodiments herein, the antigen may comprise one or more immunogenic antigen fragments of an antigen associated with an autoimmune disease. Said fragments may be the same or different. Accordingly, the antigen may comprise two antigen fragments.

In certain embodiments of the first aspect, particularly wherein the said autoimmune disease is multiple sclerosis, said antigen comprises a myelin oligodendrocyte glycoprotein (MOG) or one or more fragments thereof. Hence, said antigen may comprise one MOG fragment. In addition, said antigen may comprise more than one MOG fragment.

In one embodiment herein, the antigen comprises a MOG35-55 fragment. The antigen may also comprise more than one MOG35-55 fragment. In a preferred embodiment, the antigen comprises two MOG35-55 fragments, preferably two such fragments linked in tandem.

In the present invention, antigen fragments may be separated by spacers. Preferably, the spacers are non-immunogenic spacers. Spacers and non-immunogenic spacers are well-known to the skilled person. Preferably herein, a non-immunogenic spacer is understood as a polypeptide that does not trigger an immune response in the subject, particularly one that does not trigger same as a self-antigen triggers it. Exemplary non-immunogenic spacers herein may e.g. be designed considering of the teaching of the present examples.

In a particular embodiment herein, the antigen comprises two MOG35-55 fragments separated by spacers, particularly by spacers described elsewhere herein.

Certain embodiments herein correspond to those involving MOG35-55 fragment(s), wherein MOG34-55 fragment(s) are used instead of said MOG35-55 fragment(s).

In particular embodiments, the yeast comprises a DNA having a sequence that comprises SEQ ID NO: 3 or SEQ ID NO: 5. In a preferred embodiment herein, the yeast comprises a DNA having a sequence comprising SEQ ID NO: 5.

In particular embodiments, the yeast comprises a protein having a sequence that comprises SEQ ID NO: 1, SEQ ID NO: 2, SEQ ID NO: 4 or SEQ ID NO: 6. In a preferred embodiment herein, the yeast comprises a protein having a sequence comprising SEQ ID NO: 6. Hence, preferred antigens for use in the invention include antigens comprising SEQ ID NO: 1, SEQ ID NO: 2, SEQ ID NO: 4 or SEQ ID NO: 6, particularly SEQ ID NO: 6.

In certain embodiments, said yeast comprises both a DNA recited above and a protein recited above, such as both a DNA having a sequence comprising SEQ ID NO: 5 and a protein having a sequence comprising SEQ ID NO: 6.

In accordance with the first aspect herein, the yeast may be administered to said subject at least once a day. In certain embodiments, the yeast is administered to said subject once a day. In certain embodiments, the yeast is administered to said subject twice a day. In certain embodiments, the yeast is administered to said subject three or four times a day.

In certain embodiments, the yeast is administered to said subject over a period of at least three days. In certain embodiments, the yeast is administered to said subject over a period of at least seven days. In certain embodiments, the yeast is administered to said subject over a period of at least ten days. Accordingly, the yeast may be administered to said subject over a period of at least three days, particularly of at least seven days, especially of at least ten days. The yeast may be administered to said subject over a period of at least one week, of at least two weeks, of at least three weeks, of at least one month, of at least two months, of at least three months, of at least four months, of at least six months, of at least seven months, of at least eight months, of at least nine months, of at least ten months, of at least eleven months, of at least one year, of at least two years, etc. That is to say, the length of administration is not particularly limited. Also longer periods of administration up until permanent up to life-long administration are envisaged.

In any case, a skilled person will readily be able to determine a suitable administration time and frequency for an individual subject to be treated based on his common general knowledge and the disclosure herein.

In certain embodiments of the first aspect, the yeast may be taken up orally by said subject as part of its diet, particularly as part of its daily diet. Hence, it is e.g. considered herein that the yeast is used as a food product or part of a food product. Likewise, it is e.g. considered herein that the yeast is used as a food additive or as part of a food additive.

In certain embodiments of the first aspect, the yeast is administered in a living form. That is, it is envisaged in the present invention that living yeast (cells) are administered to the said subject.

In certain embodiments of the first aspect, the yeast is administered in an inactivated form. Hence, the yeast (cells) may have been inactivated before administration. Inactivation of yeast and yeast cells is well-known to the skilled person. A preferred mode of inactivation herein is heat-inactivation.

The amount and dosage of the yeast (cells) and antigen, respectively, used in the invention which will be effective in the prevention (and/or treatment) of a particular disorder or condition will depend on the nature of the disorder or condition. It can, however, readily be determined by the skilled person via standard clinical techniques. In addition, in vitro and/or in vivo assays may optionally be employed to help identify preferred dosage ranges of yeast (cells) and/or antigens. The precise amounts and dosages to be employed in the formulation will also depend on the severity of the disease, and will readily be decided according to the judgment of the practitioner and each patient's circumstances. Exemplary dosage ranges for oral administration herein are generally from about 1x10³ to about 1x10¹⁵ of yeast (cells) per dosage. Accordingly, in the invention, from 1x10³ to 1x10¹⁵, such as from 1x10⁵ to 1x10¹⁰, such as from 1x10⁶ to 1x10¹¹, such as from 1x10⁷ to 1x10¹², such as from 1x10⁸ to 1x10¹³, such as from 1x10⁹ to 1x10¹⁴ of yeast cells may be administered per dosage. In certain exemplary embodiments herein, 1x10⁷ to 1x10¹², particularly 1x10⁸ to 1x10¹¹, particularly 1x10⁹ to 1x10¹⁰, such as about 5x10⁹ yeast cells are administered per dosage.

Likewise, preferred dosage ranges for oral administration herein of active compound (antigen) per kilogram body weight will readily be selected by the skilled person. Besides, the skilled person may readily adjust the above amounts per dosage depending on the intended number of dosages per day or week, respectively. In any case, preferred dose ranges may also be extrapolated from dose-response curves from *in vitro* and/or *in vivo* assays.

As will readily appreciated by the skilled person, the amount and dosage, respectively, of the antigen used of the invention is preferably selected in a way that avoids or minimizes undesired effects thereof.

In the present invention, it is also envisaged that the yeast (cells) may be administered together with one or more additional agent(s). Hence, the yeast (cells) may be administered to said subject together with one or more additional agent(s), e.g. as part of a combination of agents, in a prevention and/or in a treatment of said autoimmune disease, such as in context of a combination therapy. In certain embodiments, said one or more additional agent(s) are preferably capable of affecting the severity of the autoimmune disease. According to certain embodiments, the yeast (cells) may be administered along with a given therapy of the said disease to improve said therapy, e.g. by avoiding or minimizing re-occurrence of the said disease or deterioration thereof, e.g. after improvements have been achieved by the said therapy. By such co-administration, it is also considered possible to beneficially reduce the amount of said one or more additional agent(s) to be used.

Non-limiting examples of additional agents include, but are not limited to cytokines, cytokine inhibitors (e.g. glucocorticosteriods or monoclonal antibodies), corticosteroids, cyclosporines, cytostatics (e.g. methotrexate), insulin, and monoclonal antibodies. For example, monoclonal antibodies are among the preferred examples. Also cortisone is among the preferred examples. In the case of multiple sclerosis, exemplary particular additional agents include, but are not limited to, interferon beta, corticosteroids, glatirame racetate, natalizumab, fingolimod, alemtuzumab, teriflunomid and dimethyl fumarate.

Importantly, the administration mode of the said one or more additional agent(s) is not particularly limited, particularly also as regards the administration route, and same may e.g. be administered orally, topically, parenterally e.g. subcutaneously, intramuscularly, intravascularly, etc.. Moreover, said agents may be administered as part of a composition. Said composition may comprise one or more carriers and/or excipients, that are preferably pharmaceutically acceptable, such as those described elsewhere herein.

Generally, there is no particular limitation as to the timely order of the administration of the active agents. Hence, the yeast cells may be administered before, together or after the other additional active agent(s).

In a second aspect, the present invention relates to a composition comprising yeast cells for use in the prevention of an autoimmune disease in a subject, wherein said yeast cells carry an antigen associated with an autoimmune disease on their surface, and wherein said composition is administered orally to said subject.

Generally, preferred embodiments of the second aspect correspond to those of the first aspect - and vice versa. In other words, any embodiment described herein in context with the first aspect is also envisaged as a respective embodiment in context with the second aspect - and vice versa.

Accordingly, in certain embodiments of the second aspect, the autoimmune disease may be selected from the group consisting of multiple sclerosis, rheumatoid arthritis, Crohn's disease, diabetes mellitus type 1, ulcerative colitis, autoimmune uveitis, ankylosing spondylitis, systemic lupus erythematosus, and Guillain-Barré-Syndrom. Preferably, the autoimmune disease is multiple sclerosis.

In certain embodiments of the second aspect, said subject is a mammal, particularly a human.

In certain embodiments of the second aspect, the yeast is selected from the group consisting of *Candida utilis, Pichia jadinii, Pichia stipitis, Pichia pastoris, Hansenula polymorpha,* and *Saccharomyces cerevisiae.* In other words, the said yeast cells used in the second aspect are preferably cells selected from the group consisting of *Candida utilis* cells, *Pichia jadinii* cells, *Pichia stipitis* cells, *Pichia pastoris* cells, *Hansenula polymorpha* cells, and *Saccharomyces cerevisiae* cells, more preferably from the group consisting of *Candida utilis* cells and *Pichia jadinii* cells. Accordingly, preferably herein, the yeast is *Candida utilis.* Accordingly, preferred yeast cells are *Candida utilis* cells. Also preferably herein, the yeast is *Pichia jadinii.* Accordingly, preferred yeast cells are *Pichia jadinii* cells.

In certain embodiments of the second aspect, said antigen is selected from the group consisting of myelin oligodendrocyte glycoprotein (MOG), myelin basic protein (MBP), proteolipid protein (PLP), and fragments thereof. In certain embodiments of the second aspect, said antigen comprises a protein selected from the group consisting of myelin oligodendrocyte glycoprotein (MOG), myelin basic protein (MBP), proteolipid protein (PLP), and fragments thereof.

In certain embodiments of the second aspect, the antigen is a myelin oligodendrocyte glycoprotein (MOG) or one or more fragments thereof. In certain embodiments of the second aspect, the antigen comprises a myelin oligodendrocyte glycoprotein (MOG) or one or more fragments thereof. Preferably, the antigen comprises one or more MOG fragments. Preferably, the antigen comprises one or more MOG35-55 fragments. Preferably, said antigen comprises two MOG35-55 fragments. Preferably, said fragments are separated by spacers. Preferably herein, the spacers are non-immunogenic spacers.

In certain embodiments of the second aspect, the yeast cells comprise a DNA having a sequence comprising SEQ ID NO: 5 and/or a protein having a sequence comprising SEQ ID NO: 6.

In certain embodiments of the second aspect, the composition is administered to said subject at least once a day, and/or over a period of at least three days.

The composition may be administered to said subject twice a day. The composition may be administered to said subject once a day.

The composition may be administered to said subject over a period of at least three days, particularly of at least seven days, especially of at least ten days. The composition may be administered to said subject over a period of at least one week, of at least two weeks, of at least three weeks, of at least one month, of at least two months, of at least three months, of at least four months, of at least six months, of at least seven months, of at least eight months, of at least nine months, of at least ten months, of at least eleven months, of at least one year, of at least two years, etc. That is to say, the length of administration is not particularly limited. Also longer periods of administration up until to life-long administration are envisaged.

In any case, a skilled person will readily be able to determine a suitable administration time and frequency for an individual subject to be treated based on his common general knowledge and the disclosure herein.

In certain embodiments of the second aspect, said composition may be taken up orally by said subject as part of its diet, particularly as part of its daily diet.

In certain embodiments of the second aspect, said composition may comprise living yeast cells. In alternative embodiments of the second aspect, said composition may comprise inactivated yeast cells, preferably heat-inactivated yeast cells.

In certain embodiments of the second aspect, said composition is co-administered with one or more additional agent(s). Preferably, said one or more additional agent(s) are capable of preventing and/or of affecting the severity of the said autoimmune disease.

In addition, in particular embodiments herein, the composition for use according to the second aspect is selected from a food additive and a food product.

Furthermore, in certain embodiments herein, the composition is a food additive. In other embodiments herein, the composition is a food product.

In particular embodiments herein, the composition for use according to the second aspect is a medicament

In certain embodiments herein, the composition for use according to the second aspect additionally comprises one or more carriers and/or excipients, preferably pharmaceutically acceptable carriers and/or excipients.

Preferably, pharmaceutically acceptable means that the carrier and/or excipient is approved by a regulatory agency and/or listed in a generally recognized pharmacopeia for use in animals, and more particularly, in humans.

Such carriers and/or excipients are well-known in the art and include diluents, adjuvants, wetting agents, and vehicles with which the therapeutic is administered. They may be sterile. Water is among the preferred carriers. Suitable pharmaceutical excipients include but are not limited to starch, gelatin, malt, rice, flour, chalk, silica gel, sodium stearate, glycerol monostearate, talcum, dried skim milk, glycerol, propylene, glycol, water, ethanol, glucose, lactose, sucrose, salts like sodium chloride, and the like.

In a third aspect, the invention relates to a yeast for use in a method of inducing tolerance to an antigen in a subject, wherein said yeast carries said antigen on its surface, and wherein the method comprises oral administration of said yeast to said subject. Preferably, the method is further characterized as described elsewhere herein.

As used herein, inducing tolerance includes both a full induction of tolerance and a partial induction of tolerance. Accordingly, it is sufficient herein that tolerance to a given antigen is improved in context with the said use.

Generally, preferred embodiments of the third aspect correspond to those of the first and second aspects. In other words, any embodiment described herein in context with the first and/or second aspects (and especially the first aspect) is also envisaged as a respective embodiment in context with the third aspect.

In a fourth aspect, the invention relates to a composition comprising yeast cells for use in a method of inducing tolerance to an antigen in a subject, wherein said yeast cells carry said antigen on their surface, and wherein the method comprises oral administration of said composition to said subject. Preferably, the method is further characterized as described elsewhere herein.

Generally, preferred embodiments of the fourth aspect correspond to those of the first and second aspects. In other words, any embodiment described herein in context with the first and/or second and/or third aspects (and especially the second aspect) is also envisaged as a respective embodiment in context with the fourth aspect.

In a fifth aspect, the present invention relates to methods for preventing and/or treating an autoimmune disease which comprise administering a yeast or a composition comprising yeast cells, wherein said yeast or yeast cells an antigen associated with an autoimmune disease on its/their surface, and wherein said yeast or yeast cells is/are administered orally to said subject.

Generally, preferred embodiments of the fifth aspect correspond to those of the first, second, third and fourth aspects described herein.

In a modified aspect of the present invention, the said yeast or composition comprising yeast cells is (additionally or alternatively) used in the treatment of said autoimmune disease.

Preferred embodiments of the latter aspect correspond to those of the first, second, third, fourth and fifth aspects described herein.

Generally, in the present invention, a "treatment" of an autoimmune disease may include the term as used in the art. Generally herein, it preferably relates to the treatment of subjects that currently suffer from and/or show symptoms of said disease. A treatment may correspond to amelioration of the disease and/or improvement of at least one of its symptoms. Further definitions of a treatment of an autoimmune disease correspond to those described herein in the context with a prevention of an autoimmune response. Hence, for example, in said above definitions the term "one or more symptoms are prevented" may be replaced by the term "one or more symptoms are improved". Likewise, in said above definitions the term "one or more symptoms are prevented" may be replaced by the term "one or more symptoms are ameliorated". Moreover, the term "a negative diagnosis is maintained" may be replaced by "a negative diagnosis is achieved", etc. Generally herein, a treatment may include cases where a full treatment is achieved as well as cases where a given disease is improved in any way.

Generally herein, the use of yeast (cells) in context with the present invention includes uses of said yeast (cells) in the preparation of a medicament for the prevention or treatment of an autoimmune disease. Similarly, the use of yeast (cells) in context with the present invention includes uses of said yeast (cells) in the preparation of a food or food product, particularly one that is suitable for use in the prevention or treatment of an autoimmune disease, particularly in the prevention of said disease.

Generally, in the present description and appended claims, the term "comprising" is considered to explicitly also include "consisting of". In addition, terms like "one or more" are considered to also include "one". Generally, in the present description, and in the appended claims, the singular forms "a", "an" and suchlike explicitly include plural forms, unless the context clearly specifies otherwise. Hence, reference to "a yeast" includes a plurality of such yeasts (such as a plurality of yeast cells), and such like. Said forms, however, also include the singular forms.

In general embodiments herein, and especially where not defined otherwise, the technical and scientific terms used herein have the same meaning(s) as commonly understood to a skilled person to which the invention pertains.

### Examples

The following examples are meant to further illustrate, but not limit, the invention. The examples comprise technical features, and it will be appreciated that the invention also relates to any combinations of the technical features presented in this exemplifying section.

### Example 1: Exemplary materials and methods

### Plasmid construction

For the following experiments, a publicly available *Candida utilis* strain (*Pichia jadinii*) was used, which is, for example, readily (commercially) obtainable, e.g. at the DSMZ as "DSMZ2361", or as "ATCC9950", or as "CBS5609". Plasmids used were constructed by introducing the coding sequence for the *Mus musculus* MOG₃₄₋₁₁₄ peptide between the *Nhe*I restriction sites in plasmid pCB3 (Kunigo *et al.,* 2013). The coding *MOG* sequence included the immunogenic MOG₃₅₋₅₅ epitope and a 59 amino acid long, non-immunogenic spacer sequence. The *MOG* sequence was codon optimized for the expression in *Pichia jadinii* (teleomorph (sexual) form of *Candida utilis*) based on 29 genes from the Kazusa database (www.kazusa.jp). Two plasmids, one encoding for a single MOG peptide (pCB13) (Fig. 1A) and a second encoding for a double MOG peptide (pCB10) (FIG. 1B) were constructed. These *MOG* sequences were under the control of the *C*. *utilis TDH3* promoter. The *N*-terminal secretion signal of Gas1 as well as the C-terminal GPI-signal were used to secrete the protein and link the peptide to the cell wall, respectively (FIG. 1 A + B).

Plasmids were linearized in the *TDH3* promoter region by *Sac*I for genomic integration and transformants were selected on agar plates containing 10 µg Nourseothricin/ml. Strains were designated as CBCu7 and CBCu8 carrying plasmid pCB 13 or pCB10, respectively.

### Mouse feeding protocol

To reduce the native microflora in the gut, three days before feeding 1 mg/ml tetracycline was added to the drinking water. Yeast cultures were grown for 48 h to stationary phase, cells were washed two times with PBS and the cell number was adjusted to 1x10⁹ cells/ml. Mice were fed by oral gavage with 150 µl cell suspension every 24 hours once per day.

To document *C*. *utilis* colonization, fecal pellets were collected, weighed, resuspended in 1 ml PBS, diluted and aliquots were plated out on agar plates. Colony forming units (CFU) were counted after incubation for 2 days at 30 °C.

To test whether inactivated yeast cells are able to induce oral MOG tolerance, yeast cells were grown in YPD for 48-72 h and inactivated by overnight freezing at -70 °C and thawing at 65 °C for 10 min the next day. Inactivation was verified by plating out yeast cells on agar plates.

### Immunofluorescence

Immunofluorescence was done as described earlier (Kunigo *et al.,* 2013). Briefly, stationary yeast cells were formaldehyde-fixed for 30 min, washed and resuspended in S-buffer (50 mM HEPES, 1.2 M sorbitol, pH 7.5). Glass slides were treated for 2 min with 15 µl of a 0.1 % poly-lysine solution and washed with water three times. 20 µl cells were fixed on the slide by incubation for 5 min. Unfixed cells were removed by washing four times with PBS. 20 µl of blocking solution (2 % skimmed milk powder in PBS) was added and slides were incubated for 15 min. Afterwards primary antibody (dissolved in blocking solution) was added and incubation was continued for at least 90 min. After four washing steps secondary FITC-coupled antibody was added and incubated for 90 min in the dark. The washing step was repeated and 4',6-Diamidin-2-phenylindol (DAPI) solution was added to stain chromosomal DNA. All following steps were done in the dark. After incubation for 10 min the solutions were removed by again washing slides four times with PBS. "Pro long gold antifade" solution (Invitrogen) was added according to the manufacturers protocol to prevent specimen from bleaching. Samples were observed with an Axioskop 40 (Zeiss, Cologne).

### Example 2: MOG expression / localization and plasmid stability

### MOG expression / localization

### C. utilis transformants carrying MOG plasmids were tested for MOG production. Yeast cells were grown for 72 h in YPD medium and cell extracts were separated on a SDS PAGE and MOG peptides were detected by immunoblotting using an anti-MOG₃₅₋₅₅-antibody (Aviva Systems Biology).

Results indicate that in the particular experiments, only transformants CBCu8 carrying the tandem MOG plasmid (pCB10) where initially found to express the MOG peptide, whereas in transformants CBCu7 carrying the single MOG construct (pCB13) no MOG production was observed in said initial experiments (FIG. 2).

Such findings may have various reasons, as the skilled reader will appreciate. In any case, it is emphasized in this context, however, that a skilled person is considered to be readily able to achieve successful expression in context of a given antigen associated with an autoimmune disease by routine experimentation and optionally by routine variation. Accordingly, the skilled person is considered to readily be able to produce yeast (cells) carrying any such antigen on its/their surface.

Surface localization of the MOG peptide was demonstrated by immunoflourescence microscopy. The peptide was equally distributed on the cell surface of CBCu17 yeast cells (with pCB10) (FIG. 3).

These results were verified by an immunoblot with different cell fractions of CBCu8. In the culture media no MOG signal was detected, whereas in samples containing cell wall fragments MOG was detected. In addition the MOG peptide was released from the cell wall after treatment with Zymolyase (FIG. 4). In control strains no MOG signal was detected (FIG. 4).

### Plasmid stability

In order to verify the plasmid stability of pCB10 Southern analysis was performed. Genomic DNA of three CBCu8 transformants was isolated and digested with *Kpn*I and the plasmid sequence was detected using a *TDH3p* probe. Expected sizes of plasmid bands when integrated at the *TDH3* locus were 3.9 kb and 11.3 kb, in wild- type conformation, without plasmid pCB10, a band size of 8.6 was expected (FIG. 5 A).

In all tested transformants plasmid sequences of the expected sizes of 3.9 kb and 11.3 kb were detected. Additionally, a signal at 8.6 kb corresponding to the wild-type conformation was observed (FIG. 5B). This indicates that not all *TDH3* alleles in the genome are occupied by plasmid pCB10 but at least one allele is still in wild-type conformation.

Because the chromosomal integration by homologous recombination was correct, the plasmid stability of these transformants was tested by incubating the cells for 50 generations in media either with or without Nourseothricin. The results indicate that more than 80 % of the cells were able to maintain the plasmid after non-selective incubation for 50 generations (FIG. 6). In a next experiment three Nourseothricin-resistant colonies were incubated for further 50 generations with or without Nourseothricin. After this second incubation step (in total 100 generations) the plasmid stability was further increased to 99.5 % (data not shown). One of these stable colonies (CBCu17) was used for all following mouse experiments.

### Example 3: Mouse colonization

The original purpose of the following experiments was to achieve a colonization of the mouse intestinal tract by feeding the mice with *C. utilis* CBcul7 cells and to induce oral tolerance against the MOG₃₅₋₅₅ epitope. In additional experiments an experimental autoimmune encephalomyelitis (EAE) was induced in treated and untreated mice to monitor whether CBCu17 cells are able to suppress an immune response against the MOG epitope and to weaken or even suppress an outbreak of experimental autoimmune encephalomyelitis. Since so far no comparable experiments with *C. utilis* are documented, the inventors first checked which concentration of *C*. *utilis* cells is suitable to achieve colonization. Subsequently, the survival rate of CBCu17 cells in the intestinal tract was tested. Six to eight weeks-old female C57BL/6 mice were used for this experiment. First, microorganisms in the fecal pellets of mice were counted. For this purpose, three days (d-3) before start of the *C. utilis* feeding, the fecal pellets were collected, weighed, resuspended in PBS buffer and plated out on YPD agar plates in appropriate dilutions to count colony forming units (CFU). By adding tetracycline (1 mg/ml) to the drinking water two days before the first *C. utilis* feeding the normal microbiota was expected to be reduced to enhance and facilitate growing of *C. utilis* in the intestinal tract. The fecal pellets were collected on d0 (d0: start of CBCu17 feeding) again plated out on agar plates and the number of CFUs were compared to those from d-3. It was shown that the addition of tetracycline led to a 1000-fold decrease of CFUs. Most of these CFUs were bacteria and only a small amount represented fungi (data not shown).

On day 0 (d0) a group of mice (n=5) was fed with 1x10⁷ CBCu17 cells and another group with 1x10⁸ cells. After 48 h the feeding procedure was repeated and the fecal pellet was collected and analysed every 24 h. A timescale and the results of these experiments are shown in FIG. 7.

FIG. 7 indicates that 1x10⁷ CBCu17 cells are not sufficient to pass the gastrointestinal tract, whereas in fecal pellets of mice fed with 1x10⁸ cells CBCu17 colonies were detected on the agar plates. However, after one day without feeding *C. utilis* cells disappeared in the fecal pellets. After resumption of *C. utilis* CFUs were detected one day later (d3) again on the agar plates, which then again disappeared after one day (d4) without feeding. This result showed that a two-time-feeding protocol did not result in a permanent colonization of CBCu17 in the gastrointestinal tract neither when mice fed with 1x10⁷ or with 1x10⁸ cells. In an immunoblot of three cell samples after recovery from the mouse stool the MOG protein signal was, however, still present. Therefore, passage through the mouse retained the MOG expression plasmid (data not shown).

Because a double feeding did not result in a permanent colonization of mice, in a further experiment, mice were fed repeatedly over a time period of 5 days every 24 h with 1x10⁸ cells. It was observed that *C*. *utilis* cells were in the fecal pellets but when feeding was interrupted for one day *C. utilis* cells were no longer in the collected fecal pellets (FIG. 8, d6). When mice were fed again with CBCu17 CFUs reappeared in the fecal pellets (d7). Thus, this continuous feeding with cells did not lead to a colonization of the gut in mice. To check whether *C*. *utilis* cells are in the gastrointestinal tract at all and are not excreted directly, histological samples from the duodenum, ileum, colon and caecum were analysed. CBCu17 cells were found predominantly in the caecum whereas in the other compartments no cells were identified (data not shown).

In any case, however, as also disclosed elsewhere herein, achievement of colonization is not necessary, let alone essential in the present invention.

### Example 4: Effect on autoimmune encephalomyelitis (EAE)

Furthermore, the inventors tested *Candida utilis* that expresses MOG(35-55) peptide on its surface linked to a GPI-anchor for the potential to reduce the clinical severity of the well accepted animal model of MS. The experimental autoimmune encephalomyelitis (EAE) is induced by immunization with myelin peptides, e.g. myelin oligodendrocyte glycoprotein (MOG)₃₅₋₅₅.

### Immunization:

Animal experimentation was approved by local state authorities (Landesamt für Natur, Umwelt und Verbraucherschutz Nordrhein-Westfalen). EAE was induced by immunization with MOG35-55 peptide in female C57BL/6 mice (6-8 weeks). Subcutaneous injections contained 200 µg MOG35-55 in 100 µl PBS emulsified with 100 µl complete Freund's adjuvant (CFA, Difco) containing 4 mg/ml heat inactivated Mycobacterium tuberculosis (H37Ra). Immunization was boosted with intraperitoneal injection of Pertussis toxin (500ng) at day zero and two. Clinical scoring was performed at indicated time points. 0 = no clinical signs; 1 = tail paralysis; 2 = hind limb paresis; 3 = hind limb paralysis; 4 = fore limp paresis; 5 = death.

### Treatment regimens:

In the preventive group feeding was started seven days prior to immunization with MOG35-55, whereas in the therapeutic group feeding was initiated with first clinical symptoms at day ten. C57BL/6 mice were force fed for ten subsequent days with 1.5 x 10⁸ *Candida utilis* expressing MOG peptide (CU MOG). Controls were fed with the same amount of wild type Candida or remained untreated. In initial experiments Glucose (5%) and Tetracycline (1mg/ml) was added to the drinking water to promote Candida colonization.

### T cell proliferation:

Splenocytes of immunized mice at day 15 post immunization were cultured for 72h in flat bottom 96-well plates in standard T cell medium (IMDM with 5% FCS, 2 mM L-glutamine and 50 µM 2-ME, Invitrogen) in the presence of increasing MOG 35-55 peptide concentration from 0 to 50 µg/ml. T cell proliferation was measured via [3H] thymidine incorporation during the last 24 h of a four day incubation. Liquid scintillation counting (BetaPlate1205, Perkin Elmer, Rodgau, Germany) given as counts per minute (cpm) of quadruplicate test cultures ± SEM was measured.

### Results:

In a first experiment wild type *Candida utilis* (CU ctrl) and *Candida utilis* expressing MOG peptide (CU MOG) were tested in a preventive (A) and a therapeutic (B) regimen and were compared to untreated, MOG35-55 immunized C57BL/6 mice (FIG. 10). In untreated mice first clinical symptoms became visible ten days post immunization (p.i.) with an ascending paralysis reaching maximal clinical disability at day 18 to 20 p.i.. Force feeding with the control wild type Candida strain induced comparable clinical symptoms. In contrast, oral gavage of CU MOG resulted in almost no clinical signs of EAE, it prevented the occurrence of clinical disease completely (FIG. 10, A). In this experimental setup therapeutic feeding with CU MOG did not significantly alter the clinical course of EAE (FIG. 10, B).

Thus, the experimental results clearly underline the preventive potential of CU MOG to protect against EAE - and are considered generally applicable also in the context of other autoimmune diseases.

Moreover, in view of the other positive results herein, the use of an antigen in context with the invention is also considered possible in the context of the treatment of autoimmune diseases. In particular, such use expected to be promising in the course of a treatment of autoimmune diseases, in which e.g. additional agent(s) are used, as the effect of those is expected to be improved by the present uses.

Furthermore, the following experimental results were obtained:

Next, since the preventive regimen showed a significant reduction in disease severity the experiment was repeated three times to confirm the preventive potential of *Candida utilis* expressing MOG35-55 (FIG. 11).

The reduction of the EAE severity is a result in a reduced clinical score of individual animals as well a reduced incidence. The maximal score as well as the incidence are significantly increased in the two control groups compared to the group treated with CU MOG (FIG. 12).

In line with the reduced severity of the disease, CU MOG fed animals also are less responsive to re-challenge of T cells with the cognate antigen MOG35-55 (FIG. 13). Compared to untreated mice CU MOG treated animals have a significant reduction of T cell proliferation.

Although primary experiments were performed in the presence of tetracycline and glucose with the aim of achieving colonization of CU in the microbiota of the gut, no signs of colorizations were observed in said experiments. To test the necessity for tetracycline for the preventive capacity of CU MOG the inventors compared untreated mice and CU MOG treated in the preventive paradigm (FIG. 14). Again the CU MOG treatment resulted in a reduction of the clinical score and protected against autoimmunity. Further experiments were successfully conducted in the absence of tetracycline and glucose.

To circumvent any potential disadvantages with living CU strains the preventive potential of heat inactivated CU was also tested. CU was heat inactivated at 70°C for 10 minutes after storage over night at -70°C. To confirm that heat inactivation was successful CU was cultivated for 48h in an incubator to ensure no further growing of CU after inactivation. Heat inactivated CU MOG in comparison to CU wt and CU MOG were tested in a prevention model and fed to animals comparable to experiment one and two (FIG. 15).

In line with previous experiments the feeding of CU MOG resulted in a reduction in the clinical score in comparison to the treatment with wild type CU. Most interestingly, the feeding of heat inactivated CU MOG resulted in a comparable reduction of the clinical score.

### Summary:

Accordingly, the following additional conclusions may be drawn from the experimental results:
Oral feeding of MOG35-55 expressing *Candida utilis* in a preventive paradigm can prevent CNS autoimmunity, wherein these findings are regarded generally applicable also as to other antigens and autoimmune diseases.

Without intending to be bound by theory, the protection against autoimmunity is regarded as a result of a reduced incidence and a maximum clinical score and also an antigen specific T cell proliferation.

The protection is efficient in the absence of tetracycline and glucose, indicating a colonization independent mechanism. Accordingly, in the present invention, achieving a colonization with the respective yeast (cells) is not required and clearly not essential.

Oral application of *Candida utilis* expressing MOG35-55 is not dependent of the application of living fungi, heat inactivated CU MOG contain the same preventive efficacy. Accordingly, both living and/or inactivated yeasts may be applied in the uses of the invention.

### References

Every citation of literature herein is explicitly incorporated by reference herein in its entirety.
Kunigo M., Buerth C., Tielker D. and Ernst JF (2013). Heterologous protein secretion by Candida utilis. Appl Microbiol Biotechnol (16) 7357-7368.
Weiner HL, da Cunha AP, Quintana F und Wu H (2011) Oral tolerance. Immunol Rev 241: 241-259.
Mendel I, Kerlero de Rosbo N und Ben-Nun A (1995) A myelin oligodendrocyte glycoprotein peptide induces typical chronic experimental autoimmune encephalomyelitis in H-2b mice: fine specificity and T cell receptor V beta expression of encephalitogenic T cells. Eur J Immunol 25: 1951-1959
Siebers A und Finlay BB (1996) M cells and the pathogenesis of mucosal and systemic infections. Trends Microbiol 4: 22-29

## Claims

1. A yeast for use in the prevention of an autoimmune disease in a subject,
wherein said yeast carries an antigen associated with said autoimmune disease on its surface,
and wherein said yeast is administered orally to said subject.

2. A composition comprising yeast cells for use in the prevention of an autoimmune disease in a subject,
wherein said yeast cells carry an antigen associated with said autoimmune disease on their surface,
and wherein said composition is administered orally to said subject.

3. The yeast or composition for use according to any of the preceding claims, wherein said autoimmune disease is selected from the group consisting of multiple sclerosis, rheumatoid arthritis, Crohn's disease, diabetes mellitus type 1, ulcerative colitis, autoimmune uveitis, ankylosing spondylitis, systemic lupus erythematosus, and Guillain-Barré-Syndrom,
particularly wherein said autoimmune disease is multiple sclerosis.

4. The yeast or composition for use according to any of the preceding claims, wherein said subject is a mammal, particularly a human.

5. The yeast or composition for use according to any of the preceding claims, wherein said yeast is selected from the group consisting of *Candida utilis, Pichia jadinii, Pichia stipitis, Pichia pastoris, Hansenula polymorpha, Saccharomyces cerevisiae,*
particularly wherein said yeast is *Candida utilis.*

6. The yeast or composition for use according to any of the preceding claims, wherein said autoimmune disease is multiple sclerosis,
and wherein said antigen is selected from the group consisting of myelin oligodendrocyte glycoprotein (MOG), myelin basic protein (MBP), proteolipid protein (PLP), and fragments thereof.

7. The yeast or composition for use according to any of the preceding claims, wherein said autoimmune disease is multiple sclerosis,
and wherein said antigen comprises a myelin oligodendrocyte glycoprotein (MOG) or one or more fragments thereof;
particularly wherein said antigen comprises one or more MOG fragments, especially wherein said antigen comprises one or more MOG35-55 fragments, more particularly wherein said antigen comprises two MOG35-55 fragments; and/or wherein said fragments are separated by spacers, preferably by non-immunogenic spacers.

8. The yeast or composition for use according to any of claims 6 and 7, wherein said yeast or yeast cells comprise(s) a DNA having a sequence comprising SEQ ID NO: 5 and/or a protein having a sequence comprising SEQ ID NO: 6.

9. The composition for use according to any of claims 2 to 8, wherein said composition is selected from a food additive and a food product,
particularly wherein said composition is a food additive,
especially wherein said composition is a food product.

10. The composition for use according to any of claims 2 to 8, wherein the composition is a medicament
and/or
wherein said composition additionally comprises one or more carriers and/or excipients.

11. The yeast or composition for use according to any of the preceding claims, wherein said yeast or composition is administered to said subject
i) at least once a day,
particularly wherein said yeast or composition is administered to said subject once or twice a day, especially wherein said yeast or composition is administered to said subject once a day;
and/or
ii) over a period of at least three days, particularly of at least seven days, especially of at least ten days.

12. The yeast or composition for use according to any of the preceding claims, wherein said yeast or composition is taken up orally by said subject as part of its diet, particularly as part of its daily diet.

13. The yeast or composition for use according to any of the preceding claims, wherein said yeast is administered in a living form or wherein said yeast is administered in an inactivated form,
particularly wherein said yeast is administered in an inactivated form,
especially wherein said yeast is administered in a heat-inactivated form.

14. The yeast or composition for use according to any of the preceding claims, wherein one or more additional agent(s) are administered to said subject,
particularly wherein said one or more additional agent(s) are capable of affecting the severity of the said autoimmune disease.

15. A yeast or a composition comprising yeast cells for use in a method of inducing tolerance to an antigen in a subject,
wherein said yeast or yeast cells carries/carry said antigen on its/their surface,
and wherein the method comprises oral administration of said yeast or composition to said subject,
particularly wherein the method is further **characterized in** accordance with any one of claims 3 to 14.


SEQUENCE LISTING

<110>  Heinrich-Heine-Universität Düsseldorf

<120>  Induction of Immunological Tolerance Using Yeast

<130>  P69186EP

<160>  6

<170>  PatentIn version 3.5

<210>  1
<211>  160
<212>  PRT
<213>  Artificial Sequence

<220>
<223>  Amino acid sequence of the Gas1-Mog fusion protein including
       Gas1-Gpi sequence

<400>  1

Met Arg Phe Asn Trp Ala Leu Ala Ser Ala Phe Ala Gly Val Ala Leu
1               5                   10                  15

Ala Asn Gln Ala Arg Ala Ser Gly Met Glu Val Gly Trp Tyr Arg Ser
            20                  25                  30

Pro Phe Ser Arg Val Val His Leu Tyr Arg Asn Gly Lys Asp Gln Asp
        35                  40                  45

Ala Glu Gln Ala Pro Glu Tyr Arg Gly Arg Thr Glu Leu Leu Lys Glu
    50                  55                  60

Thr Ile Ser Glu Gly Lys Val Thr Leu Arg Ile Gln Asn Val Arg Phe
65                  70                  75                  80

Ser Asp Glu Gly Gly Tyr Thr Cys Phe Phe Arg Asp His Ser Tyr Gln
                85                  90                  95

Glu Glu Ala Ala Met Glu Leu Lys Val Glu Asp Ala Ser Ser Thr Ser
            100                 105                 110

Ser Ser Thr Ala Ser Ala Ser Ala Ser Ala Ser Ser Thr Gly Lys Ser
        115                 120                 125

Ser Ala Gly Ala Val Asn Pro Val Gly Ala Ser Asn Val Ala Leu Leu
    130                 135                 140

Met Thr Ile Gly Thr Phe Phe Thr Val Ser Met Gly Ala Phe Leu Phe
145                 150                 155                 160

<210>  2
<211>  246
<212>  PRT
<213>  Artificial Sequence

<220>
<223>  Amino acid sequence of Gas1-Mog-Mog fusion protein including
       Gas1-Gpi sequence

<400>  2

Met Arg Phe Asn Trp Ala Leu Ala Ser Ala Phe Ala Gly Val Ala Leu
1               5                   10                  15

Ala Asn Gln Ala Arg Ala Ser Gly Met Glu Val Gly Trp Tyr Arg Ser
            20                  25                  30

Pro Phe Ser Arg Val Val His Leu Tyr Arg Asn Gly Lys Asp Gln Asp
        35                  40                  45

Ala Glu Gln Ala Pro Glu Tyr Arg Gly Arg Thr Glu Leu Leu Lys Glu
    50                  55                  60

Thr Ile Ser Glu Gly Lys Val Thr Leu Arg Ile Gln Asn Val Arg Phe
65                  70                  75                  80

Ser Asp Glu Gly Gly Tyr Thr Cys Phe Phe Arg Asp His Ser Tyr Gln
                85                  90                  95

Glu Glu Ala Ala Met Glu Leu Lys Val Glu Asp Ala Ser Gly Met Glu
            100                 105                 110

Val Gly Trp Tyr Arg Ser Pro Phe Ser Arg Val Val His Leu Tyr Arg
        115                 120                 125

Asn Gly Lys Asp Gln Asp Ala Glu Gln Ala Pro Glu Tyr Arg Gly Arg
    130                 135                 140

Thr Glu Leu Leu Lys Glu Thr Ile Ser Glu Gly Lys Val Thr Leu Arg
145                 150                 155                 160

Ile Gln Asn Val Arg Phe Ser Asp Glu Gly Gly Tyr Thr Cys Phe Phe
                165                 170                 175

Arg Asp His Ser Tyr Gln Glu Glu Ala Ala Met Glu Leu Lys Val Glu
            180                 185                 190

Asp Ala Ser Ser Thr Ser Ser Ser Thr Ala Ser Ala Ser Ala Ser Ala
        195                 200                 205

Ser Ser Thr Gly Lys Ser Ser Ala Gly Ala Val Asn Pro Val Gly Ala
    210                 215                 220

Ser Asn Val Ala Leu Leu Met Thr Ile Gly Thr Phe Phe Thr Val Ser
225                 230                 235                 240

Met Gly Ala Phe Leu Phe
                245

<210>  3
<211>  264
<212>  DNA
<213>  Artificial Sequence

<220>
<223>  Exemplary DNA sequence including MOG35-55 sequence
       (Single-MOG-DNA sequence)

<220>
<221>  CDS
<222>  (1)..(264)

<400>  3
gct agc ggt atg gaa gtt ggt tgg tac aga tct cca ttc tct aga gtt      48
Ala Ser Gly Met Glu Val Gly Trp Tyr Arg Ser Pro Phe Ser Arg Val
1               5                   10                  15

gtt cac ttg tac aga aac ggt aag gat caa gat gct gag caa gct cca      96
Val His Leu Tyr Arg Asn Gly Lys Asp Gln Asp Ala Glu Gln Ala Pro
            20                  25                  30

gag tac aga ggt aga acc gag ttg ttg aag gaa acc atc tct gag ggt      144
Glu Tyr Arg Gly Arg Thr Glu Leu Leu Lys Glu Thr Ile Ser Glu Gly
        35                  40                  45

aag gtt acc ttg aga atc caa aac gtt aga ttc tct gat gag ggt ggt      192
Lys Val Thr Leu Arg Ile Gln Asn Val Arg Phe Ser Asp Glu Gly Gly
    50                  55                  60

tac acc tgt ttc ttc aga gat cac tct tac caa gag gaa gct gct atg      240
Tyr Thr Cys Phe Phe Arg Asp His Ser Tyr Gln Glu Glu Ala Ala Met
65                  70                  75                  80

gaa ttg aag gtt gag gat gct agc                                      264
Glu Leu Lys Val Glu Asp Ala Ser
                85

<210>  4
<211>  88
<212>  PRT
<213>  Artificial Sequence

<220>
<223>  Synthetic Construct

<400>  4

Ala Ser Gly Met Glu Val Gly Trp Tyr Arg Ser Pro Phe Ser Arg Val

1               5                   10                  15

Val His Leu Tyr Arg Asn Gly Lys Asp Gln Asp Ala Glu Gln Ala Pro
            20                  25                  30

Glu Tyr Arg Gly Arg Thr Glu Leu Leu Lys Glu Thr Ile Ser Glu Gly
        35                  40                  45

Lys Val Thr Leu Arg Ile Gln Asn Val Arg Phe Ser Asp Glu Gly Gly
    50                  55                  60

Tyr Thr Cys Phe Phe Arg Asp His Ser Tyr Gln Glu Glu Ala Ala Met
65                  70                  75                  80

Glu Leu Lys Val Glu Asp Ala Ser
                85

<210>  5
<211>  522
<212>  DNA
<213>  Artificial Sequence

<220>
<223>  Exemplary DNA sequence including MOG35-55 sequence
       (Tandem-MOG-DNA sequence)

<220>
<221>  CDS
<222>  (1)..(522)

<400>  5
gct agc ggt atg gaa gtt ggt tgg tac aga tct cca ttc tct aga gtt      48
Ala Ser Gly Met Glu Val Gly Trp Tyr Arg Ser Pro Phe Ser Arg Val
1               5                   10                  15

gtt cac ttg tac aga aac ggt aag gat caa gat gct gag caa gct cca      96
Val His Leu Tyr Arg Asn Gly Lys Asp Gln Asp Ala Glu Gln Ala Pro
            20                  25                  30

gag tac aga ggt aga acc gag ttg ttg aag gaa acc atc tct gag ggt      144
Glu Tyr Arg Gly Arg Thr Glu Leu Leu Lys Glu Thr Ile Ser Glu Gly
        35                  40                  45

aag gtt acc ttg aga atc caa aac gtt aga ttc tct gat gag ggt ggt      192
Lys Val Thr Leu Arg Ile Gln Asn Val Arg Phe Ser Asp Glu Gly Gly
    50                  55                  60

tac acc tgt ttc ttc aga gat cac tct tac caa gag gaa gct gct atg      240
Tyr Thr Cys Phe Phe Arg Asp His Ser Tyr Gln Glu Glu Ala Ala Met
65                  70                  75                  80

gaa ttg aag gtt gag gat gct agc ggt atg gaa gtt ggt tgg tac aga      288
Glu Leu Lys Val Glu Asp Ala Ser Gly Met Glu Val Gly Trp Tyr Arg
                85                  90                  95

tct cca ttc tct aga gtt gtt cac ttg tac aga aac ggt aag gat caa      336

Ser Pro Phe Ser Arg Val Val His Leu Tyr Arg Asn Gly Lys Asp Gln
            100                 105                 110

gat gct gag caa gct cca gag tac aga ggt aga acc gag ttg ttg aag         384
Asp Ala Glu Gln Ala Pro Glu Tyr Arg Gly Arg Thr Glu Leu Leu Lys
        115                 120                 125

gaa acc atc tct gag ggt aag gtt acc ttg aga atc caa aac gtt aga         432
Glu Thr Ile Ser Glu Gly Lys Val Thr Leu Arg Ile Gln Asn Val Arg
    130                 135                 140

ttc tct gat gag ggt ggt tac acc tgt ttc ttc aga gat cac tct tac         480
Phe Ser Asp Glu Gly Gly Tyr Thr Cys Phe Phe Arg Asp His Ser Tyr
145                 150                 155                 160

caa gag gaa gct gct atg gaa ttg aag gtt gag gat gct agc                 522
Gln Glu Glu Ala Ala Met Glu Leu Lys Val Glu Asp Ala Ser
                165                 170


<210>  6
<211>  174
<212>  PRT
<213>  Artificial Sequence

<220>
<223>  Synthetic Construct

<400>  6

Ala Ser Gly Met Glu Val Gly Trp Tyr Arg Ser Pro Phe Ser Arg Val
1               5                   10                  15


Val His Leu Tyr Arg Asn Gly Lys Asp Gln Asp Ala Glu Gln Ala Pro
            20                  25                  30


Glu Tyr Arg Gly Arg Thr Glu Leu Leu Lys Glu Thr Ile Ser Glu Gly
        35                  40                  45


Lys Val Thr Leu Arg Ile Gln Asn Val Arg Phe Ser Asp Glu Gly Gly
    50                  55                  60


Tyr Thr Cys Phe Phe Arg Asp His Ser Tyr Gln Glu Glu Ala Ala Met
65                  70                  75                  80


Glu Leu Lys Val Glu Asp Ala Ser Gly Met Glu Val Gly Trp Tyr Arg
                85                  90                  95


Ser Pro Phe Ser Arg Val Val His Leu Tyr Arg Asn Gly Lys Asp Gln
            100                 105                 110


Asp Ala Glu Gln Ala Pro Glu Tyr Arg Gly Arg Thr Glu Leu Leu Lys
        115                 120                 125


Glu Thr Ile Ser Glu Gly Lys Val Thr Leu Arg Ile Gln Asn Val Arg

    130                 135                 140

Phe Ser Asp Glu Gly Gly Tyr Thr Cys Phe Phe Arg Asp His Ser Tyr
145                 150                 155                 160

Gln Glu Glu Ala Ala Met Glu Leu Lys Val Glu Asp Ala Ser
                165                 170